# FASCICULE DE BREVET EUROPEEN

(11) **EP 1 550 656 B1**
(45) Date de publication et mention de la délivrance du brevet: **06.05.2009**
(21) Numéro de dépôt: 04292749.1
(22) Date de dépôt: 22.11.2004
(51) Int. Cl.: C07D 487/04, A61K 8/49, A61Q 5/10

(54) **Dérivés de 4-5-diamino-N,N-dihydro-pyrazol-3-one condensé, utilisés pour la teinture des fibres keratiniques**
Kondensierte 4-5-diamino-N,N-dihydro-pyrazol-3-on-Derivate und deren Verwendung als Färbemittel für keratinfasern
fused 4-5-diamino-n,n-dihydro-pyrazol-3-one derivatives for use in composition for dyeing keratin fibres

(30) Priorité: 01.12.2003 FR 0350950; 18.02.2004 FR 0450297
(43) Date de publication de la demande: 06.07.2005
(60) Demande divisionnaire: 06122216.2
(73) Titulaire: L'ORÉAL, 75008 Paris (FR)
(72) Inventeur: Vidal, Laurent, 75013 Paris (FR); Fadli, Aziz, 77500 Chelles (FR)
(74) Mandataire: Fevrier, Murielle Françoise E.

(56) Documents cités:
- EP-A- 0 873 745
- EP-A- 1 250 909
- CH-A- 421 343
- DE-A- 1 959 009
- US-A- 3 012 884
- US-A- 4 128 425
- US-A- 4 886 736

## Description

L'invention a pour objet une composition pour la teinture des fibres kératiniques, et en particulier des fibres kératiniques humaines telles que les cheveux, comprenant au moins un dérivé de diamino-N,N-dihydropyrazolone ou un de ses sels d'addition à titre de base d'oxydation et un procédé la mettant en oeuvre. Elle a de même pour objet des dérivés d'amino-N,N-dihydropyrazolone ainsi que des dérivés de diamino-N,N-dihydropyrazolone ou un de ses sels d'addition en tant que tels et leur obtention.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4-triazoles, des dérivés de pyrazolo[3,2-c]-1,2,4-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

L'utilisation de base d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

L'utilisation de ces bases à pH neutre est de plus inefficace pour atteindre une gamme de nuances variées, en particuliers pour les nuances chaudes.

Il a été déjà proposé dans le brevet DE3843892 d'utiliser certains dérivés de diaminopyrazoles, notamment pour les nuances rouge à rouge cuivrée. Cependant, cette proposition ne permet pas d'atteindre de bonnes propriétés de chromaticité, de résistance aux agents extérieurs telle que le lavage et la lumière. De plus, l'étendue de la gamme des nuances est limitée.

Par ailleurs, il est connu de la demande de brevet EP 1 250 909 d'utiliser un dérivé de pyrazole, en particulier le 3,4-diamino-5-hydroxypyrazole, à titre de colorant d'oxydation pour la teinture des cheveux.

Il est également connu de la demande de brevet EP 0 873 745 d'utiliser le 1-phényl-3-carboxyamido-4-amino-pyrazol-5-one à titre de base d'oxydation et le 1-phényl-3-méthyl-pyrazol-5-one à titre de capteur pour la coloration des fibres kératiniques.

Or, la demanderesse a découvert de façon totalement surprenante, que de nouveaux composés de diamino-N,N-dihydro-pyrazolone de formule (I) conviennent pour une utilisation comme précurseurs de coloration d'oxydation et permettent d'obtenir une coloration aux nuances variées, puissante, chromatique, esthétique, peu sélective et résistant bien aux diverses agressions que peuvent subir les cheveux tels que les shampooings, la lumière, la sueur et les déformations permanentes.

La demanderesse a également découvert de façon surprenante que les colorations obtenues à pH neutre sont intenses.

La présente invention a donc pour objet une composition tinctoriale des fibres kératiniques comprenant, dans un milieu de teinture approprié, à titre de base d'oxydation, au moins un dérivé de la diamino-N,N-dihydro-pyrazolone de formule (I) ou l'un de ses sels d'addition : 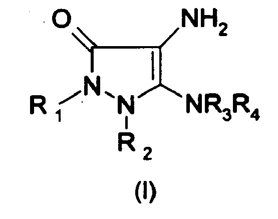 dans laquelle :
R₁ et R₂ forment avec les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R₃ et R₄, identiques ou différents, représentent :
   - un radical alkyle en C₁-C₆ linéaire ou ramifié éventuellement substitué par un ou plusieurs radicaux choisi dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un groupe (C₁-C₄)alkyle, un hydroxy, un alcoxy en C₁-C₂, un amino, un (di)alkyl(C₁-C₂)amino;
   - un radical aryle éventuellement substitué par un ou plusieurs (C₁-C₄)alkyle, hydroxy, alcoxy en C₁-C₂, amino, (di)alkyl(C₁-C₂)amino ;
   - un radical hétéroaryle à 5 ou 6 chaînons, éventuellement substitué par un ou plusieurs radicaux choisis parmi (C₁-C₄)alkyle, (C₁-C₂)alcoxy;
R₃ et R₄ peuvent représenter également un atome d'hydrogène ;
R₅, R₆ et R₇, identiques ou différents, représentent un atome d'hydrogène ; un radical alkyle linéaire ou ramifié en C₁-C₄ éventuellement substitué par un ou plusieurs radicaux choisis dans le groupe constitué par un hydroxy, un alcoxy en C₁-C₂, un carboxamido CONR₈R₉, un sulfonyle SO₂R₈, un aryle éventuellement substitué par un (C₁-C₄)alkyle, un hydroxy, un alcoxy en C₁-C₂, un amino, un (di)alkyl(C₁-C₂)amino ; un aryle éventuellement substitué par un (C₁-C₄)alkyle , un hydroxy, un alcoxy en C₁-C₂, un amino, un (di)alkyl(C₁-C₂)amino ;
R₆ et R₇, identiques ou différents, peuvent représenter également un radical carboxamido CONR₈R₉ ; un sulfonyle SO₂R₈ ;
R₈ et R₉, identiques ou différents, représentent un atome d'hydrogène ; radical alkyle en C₁-C₄ linéaire ou ramifié éventuellement substitués par un ou plusieurs hydroxy, alcoxy en C₁-C₂;
R₃ et R₄ peuvent former avec l'atome d'azote auquel ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R₃ et R₄ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué.

La présente invention permet en particulier d'obtenir une coloration des fibres kératiniques tenace, résistante à la lumière et au lavage.

Un autre objet de l'invention est un procédé de teinture des fibres kératiniques mettant en oeuvre la composition de la présente invention, ainsi que l'utilisation de cette composition pour la teinture des fibres kératiniques.

L'invention a aussi pour objet de nouveaux dérivés d'amino-N-N-dihydro-pyrazolone, ainsi que des dérivés de diamino-N,N-dihydro-pyrazolone.

L'invention concerne enfin de nouveaux procédés de synthèse de ces dérivés de (di)amino-N,N-dihydropyrazolone de formules (I') et (I'') ou leurs sels d'addition.

Comme indiqué précédemment, la composition comprend au moins un dérivé de diamino-N,N-dihydro-pyrazolone de formule (I) ou un de ses sels d'addition.

Selon un mode de réalisation, les radicaux R₁ et R₂ forment ensemble avec les atomes d'azotes auxquels ils sont rattachés, un cycle à 5 ou 6 chaînons, saturé ou insaturé, éventuellement substitué.

De préférence, les radicaux R₁ et R₂ forment ensemble avec les atomes d'azotes auxquels ils sont rattachés, un cycle pyrazolidine, pyridazolidine, éventuellement substitué par un radical alkyle en C₁-C₄, un hydroxy, un (C₁-C₂)alcoxy, un carboxy, un carboxamido, un amino, un (di)alkyl(C₁-C₂)amino.

De manière encore plus avantageuse, les radicaux R₁ et R₂ forment ensemble avec les atomes d'azotes auxquels ils sont rattachés, un cycle pyrazolidine, pyridazolidine.

En ce qui concerne les radicaux R₃ et R₄, ces derniers, identiques ou différents, sont plus particulièrement choisis parmi un atome d'hydrogène ; un radical alkyle en C₁-C₄ linéaire ou ramifié, éventuellement substitué par un ou plusieurs hydroxy, (C₁-C₂)alcoxy, amino, un (di)alkyl(C₁-C₂)amino ; un radical phényle éventuellement substitué par un radical hydroxy, amino, (C₁-C₂)alcoxy.

De préférence, les radicaux R₃ et R₄, identiques ou non, sont choisis parmi un atome d'hydrogène, un méthyle, éthyle, isopropyle, 2-hydroxyéthyle, 3-hydroxypropyle, 2-hydroxypropyle, 2-carboxyéthyle. Selon un mode de réalisation particulier, les radicaux R₃ et R₄, représentent un atome d'hydrogène.

Selon un autre mode de réalisation, les radicaux R₃ et R₄ forment ensemble avec l'atome d'azote auquel ils sont rattachés, un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl(C₁-C₂)amino, carboxy, carboxamido, alkyle en C₁-C₄ éventuellement substitué par un ou plusieurs hydroxy, amino, (di)alkylamino en C₁-C₂.

Plus particulièrement, les radicaux R₃ et R₄ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino- pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

De préférence, les radicaux R₃ et R₄ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthyl homopipérazine, la N β-hydroxyéthylhomopipérazine.

Conformément à un mode de réalisation encore plus préféré de l'invention, les radicaux R₃ et R₄ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

Les composés de formule (I) peuvent être éventuellement salifiés par des acides minéraux forts tels que par exemple HCl, HBr, Hl, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isopropanol.

A titre d'exemples de dérivés de formule (I), on peut citer les composés présentés ci-dessous ou leurs sels d'addition.
2,3-diamino-6,7-dihydro-1H,5H-pyrazolo[1,2-a]pyrazol-1-one
2-amino-3-méthylamino-6,7-dihydro-1H,5H-pyrazolo[1,2-a]pyrazol-1-one
2-amino-3-diméthylamino-6,7-dihydro-1H,5H-pyrazolo[1,2-a]pyrazol-1-one
2-amino-3-éthylamino-6,7-dihydro-1H,5H-pyrazolo[1,2-a]pyrazol-1-one
2-amino-3-isopropylamino-6,7-dihydro-1H,5H-pyrazolo[1,2-a]pyrazol-1-one
2-amino-3-(2-hydroxyéthyl)amino-6,7-dihydro-1H,5H-pyrazolo[1,2-a]pyrazol-1-one
2-amino-3-(2-hydroxypropyl)amino-6,7-dihydro-1H,5H-pyrazolo[1,2-a]pyrazol-1-one
2-amino-3-bis(2-hydroxyéthyl)amino₋6,7-dihydro-1H,5H-pyrazolo[1,2-a]pyrazol-1-one

2-amino-3-(pyrrolidin-1-yl)-6,7-dihydro-1H,5H-pyrazolo[1,2-a]pyrazol-1-one
2-amino-3-(3-hydroxy-pyrrolidin-1-yl)-6,7-dihydro-1H,5H-pyrazolo[1,2-a]pyrazol-1-one
2-amino-3-(pipéridin-1-yl)-6,7-dihydro-1H,5H-pyrazolo[1,2-a]pyrazol-1-one

2,3-diamino-6-hydroxy-6,7-dihydro-1H,5H-pyrazolo[1,2-a]pyrazol-1-one
2,3-diamino-6-méthyl-6,7-dihydro-1H,5H-pyrazolo[1,2-a]pyrazol-1-one
2,3-diamino-6-diméthyl-6,7-dihydro-1H,5H-pyrazolo[1,2-a]pyrazol-1-one

2,3-diamino-5,6,7,8-tétrahydro-1H,6H-pyridazino[1,2-a]pyrazol-1-one
2,3-iamino-5,8-dihydro-1H,6H-pyridazino[1,2-a]pyrazol-1-one

2,3-Diamino-6-hydroxy-6,7-dihydro-5H-pyrazolo[1,2-a]pyrazol-1-one
dont certains sont figurés ci-dessous pour illustrer les noms par des structures chimiques :

| | |
|---|---|
| 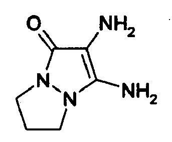 | 2,3-diamino-6,7-dihydro-1H,5H-pyrazolo[1,2-a]pyrazol-1-one |
| 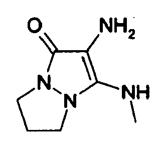 | 2-Amino-3-méthylamino-6,7-dihydro-1H,5H-pyrazolo[1,2-a]pyrazol-1-one |
| 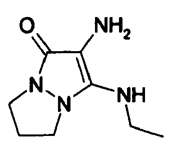 | 2-Amino-3-éthylamino-6,7-dihydro-1H,5H-pyrazolo[1,2-a]pyrazol-1-one |
| 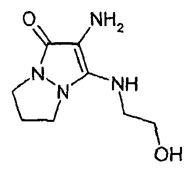 | 2-amino-3-(2-hydroxyéthyl)amino-6,7-dihydro-1H,5H-pyrazolo[1,2-a]pyrazol-1-one |
| 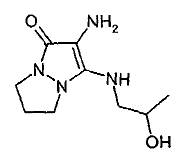 | 2-amino-3-(2-hydroxypropyl)amino-6,7-dihydro-1H,5H-pyrazolo[1,2-a]pyrazol-1-one |
| 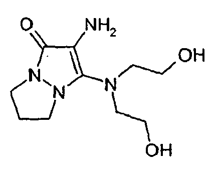 | 2-amino-3-bis(2-hydroxyéthyl)amino-6,7-dihydro-1H,5H-pyrazolo[1,2-a]pyrazol-1-one |
| 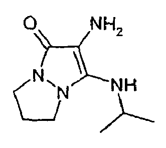 | 2-amino-3-isopropylamino-6,7-dihydro-1H,5H-pyrazolo[1,2-a]pyrazol-1-one |
| 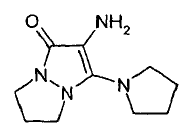 | 2-amino-3-(pyrrolidin-1-yl)-6,7-dihydro-1H,5H-pyrazolo[1,2-a]pyrazol-1-one |
| 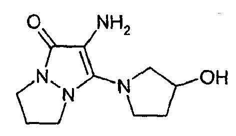 | 2-amino-3-(3-hydroxy-pyrrolidin-1-yl)-6,7-dihydro-1H,5H-pyrazolo[1,2-a]pyrazol-1-one |
| 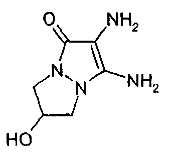 | 2,3-diamino-6-hydroxy-6,7-dihydro-1H,5H-pyrazolo[1,2-a]pyrazol-1-one |
| 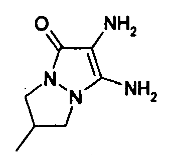 | 2,3-diamino-6-méthyl-6,7-dihydro-1H,5H-pyrazolo[1,2-a]pyrazol-1-one |
| 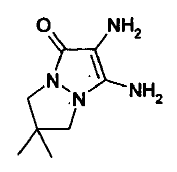 | 2,3-diamino-6-diméthyl-6,7-dihydro-1H,5H-pyrazolo[1,2-a]pyrazol-1-one |
| 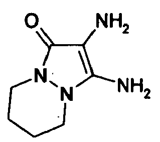 | 2,3-diamino-5,6,7,8-tétrahydro-1H,6H-pyridazino[1,2-a]pyrazol-1-one |
| 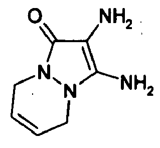 | 2,3-diamino-5,8-dihydro-1 H,6H-pyridazino[1,2-a]pyrazol-1-one |

Parmi ces composés, les dérivés de diamino-N,N-dihydropyrazolone de formule (I) ou leurs sels d'addition, particulièrement préférés sont les :
2,3-Diamino-6,7-dihydro-1H,5H-pyrazolo[1,2-a]pyrazol-1-one
2-Amino-3-éthylamino-6,7-dihydro-1H,5H-pyrazolo[1,2-a]pyrazol-1-one
2-Amino-3-isopropylamino-6,7-dihydro-1H,5H-pyrazolo[1,2-a]pyrazol-1-one
2-Amino-3-(pyrrolidin-1-yl)-6,7-dihydro-1H,5H-pyrazolo[1,2-a]pyrazol-1-one
2-amino-3-(2-hydroxyéthyl)amino-6,7-dihydro-1H,5H-pyrazolo[1,2-a]pyrazol-1-one
2-amino-3-diméthylamino-6,7-dihydro-1H,5H-pyrazolo[1,2-a]pyrazol-1-one
2,3-diamino-5,6,7,8-tétrahydro-1H,6H-pyridazino[1,2-a]pyrazol-1-one

La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis précédemment et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 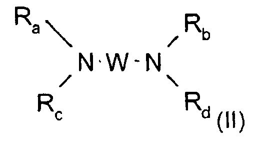 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

La présente invention a également pour objet l'utilisation pour la teinture d'oxydation des fibres kératiniques, et en particulier des fibres kératiniques humaines telles que les cheveux, d'un dérivé de diamino-N,N-dihydropyrazolone de formule (I) ou d'un de ses sels d'addition tel que défini précédemment.

Constituent également un autre objet de la présente invention, les dérivés d'amino-N,N-dihydro-pyrazolone de formule (I') suivante, et leurs sels d'addition : 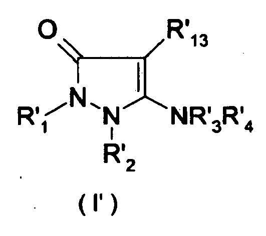 formule dans laquelle :
R'₁, R'₂, R'₃ et R'₄ ont respectivement les mêmes significations que R₁, R₂, R₃ et R₄, sous réserve que
   - R'₁₃ ne représente pas un groupe Ar-N=N- lorsque R'₃ et R'₄ représentent simultanément un atome d'hydrogène.
R'₁₃ représente un groupe nitro, nitroso ou arylazo Ar-N=N-, le radical aryle Ar étant éventuellement substitué par un radical alkyle en C₁-C₄, amino, (di)alkyl(C₁-C₄)amino, alcoxy en C₁-C₂, sulfonique, carboxy, halogène.

Tout ce qui a été indiqué précédemment à propos des définitions préférées des radicaux R₁, R₂, R₃ et R₄, est valable pour R'₁, R'₂, R'₃ et R'₄ et ne sera pas repris dans cette partie du texte.

Un autre objet de la présente invention est également constitué par les dérivés de diamino-N,N-dihydro-pyrazolone de formule (I") suivante, et leurs sels d'addition : 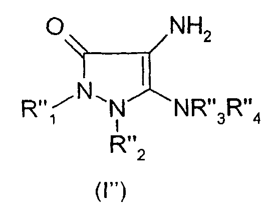 formule dans laquelle R"₁, R"₂, R"₃ et R"₄ ont les mêmes significations que celles indiquées auparavant dans le texte pour R'₁, R'₂, R'₃, R'₄.

Là encore, tout ce qui a été indiqué précédemment à propos des définitions préférées des radicaux R'₁, R'₂, R'₃ et R'₄, est valable pour R"₁, R"₂, R"₃ et R"₄ et ne sera pas repris dans cette partie du texte.

Les dérivés d'amino-N,N-dihydropyrazolone et de diamino-N,N-dihydropyrazolone conformes à l'invention et dont les radicaux R'₃ et R'₄ d'une part et R"₃ et R"₄ d'autre part représentent un atome d'hydrogène peuvent être obtenus à partir d'intermédiaires et de voies de synthèse décrites dans la littérature et notamment dans les références suivantes : J. Het. Chem., 2001, 38(3), 613-616 , Helvetica Chimica Acta, 1950, 33, 1183-1194, J.Org.Chem., 23, 2029 (1958), J.Am.Chem.Soc., 73, 3240 (1951), J.Am.Chem.Soc., 84, 590 (1962), Justus Liebig Ann.Chem., 686, 134 (1965), Tetrahedron. Lett., 31, 2859-2862 (1973), les brevets US4128425 et US 2841584 et les références citées.

Suivant ces références, les composés de formule (I) ayant les radicaux R₃ et R₄ égaux à des atomes d'hydrogène peuvent être obtenus à partir de la voie de synthèse représentée sur le schéma A ci-dessous :

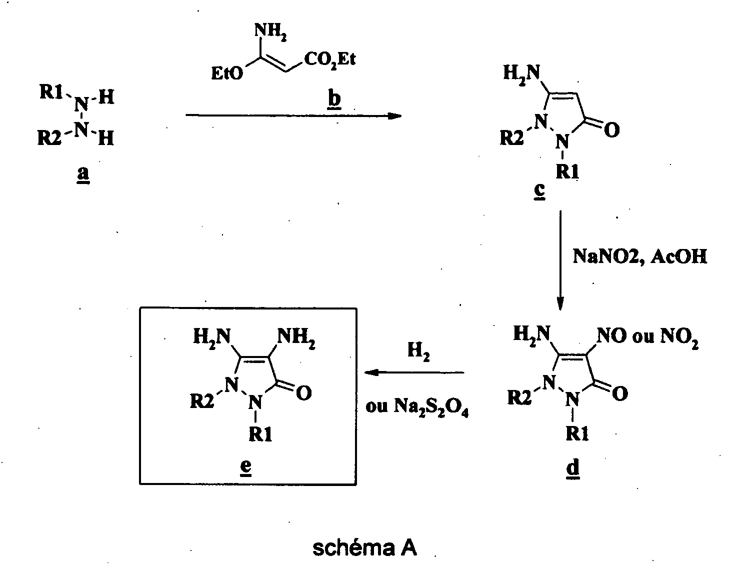

Les composés conformes à l'invention et dont les radicaux R₁ et R₂ forment ensemble un cycle à 5 chaînons et dont les radicaux R₃ et R₄ représentent des atomes d'hydrogène peuvent être obtenus en s'inspirant de la méthode décrite dans J. Het. Chem., 2001, 38(3), 613-616 (schéma D) :

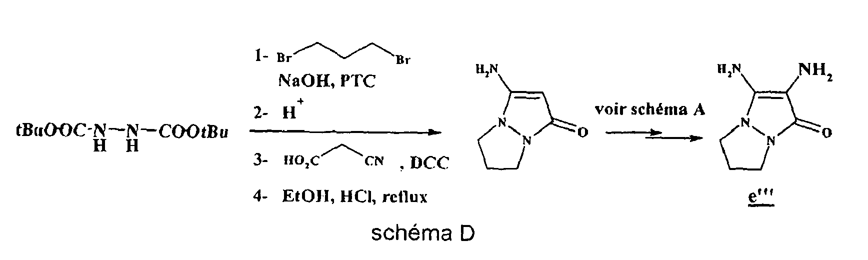

Selon un nouveau procédé, les composés de formule (I) peuvent être obtenus selon la synthèse illustrée dans le schéma E :

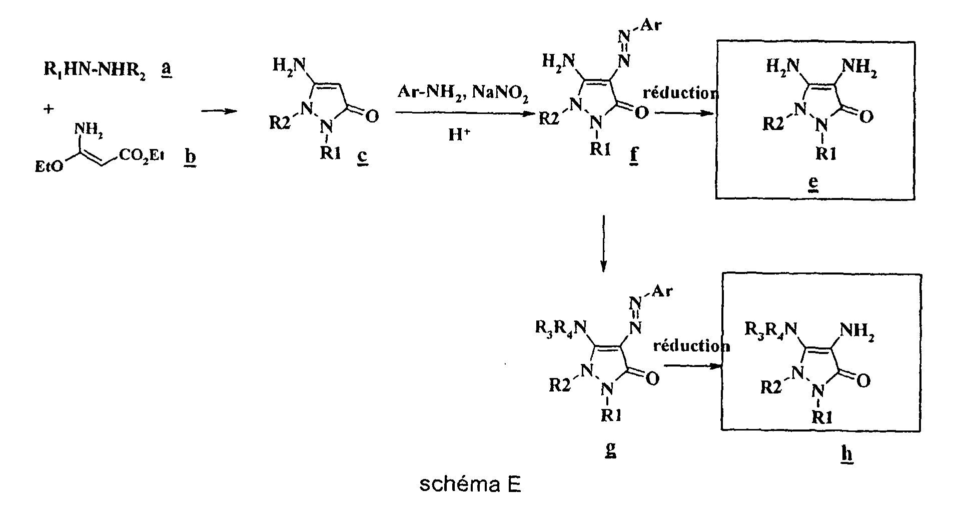

Selon ce nouveau procédé, on met en oeuvre les étapes suivantes :
a) étape 1 :on fait réagir un composé **a**

   **R₁HN-NHR₂** **a**

   avec un composé **b** : 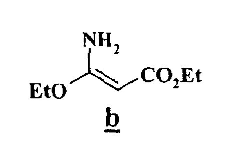 pour obtenir un composé 5-amino-1,2-dihydro-pyrazol-3-one **c** : 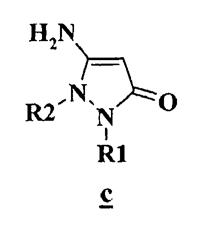
b) étape 2 : on fait réagir le dérivé **c** ainsi obtenu un sel d'aryldiazonium (Ar-NH₂, NaNO₂, H⁺) pour obtenir un composé azoïque **f** : 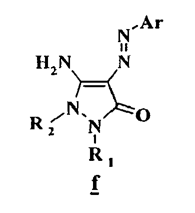
c) étape 3 : on effectue éventuellement une étape de fonctionalisation du groupement amine primaire du composé azoïque résultant **f** pour obtenir un composé **g** suivant : 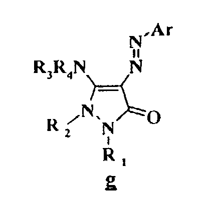
d) étape 4 : on effectue une réaction de réduction du composé azoïque **f** ou **g** pour obtenir, respectivement, un composé **e** ou **h** aminé :

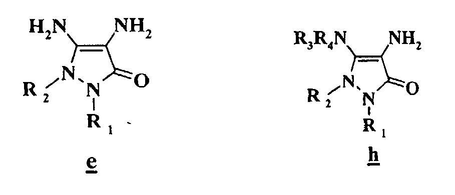

L'étape éventuelle de fonctionalisation du groupement amine primaire en position 5 en amine secondaire et tertiaire NR₃R₄, pour obtenir les composés **g,** est réalisée selon les méthodes classiques de synthèse organique (halogénure d'alkyle, O-sulfonate d'alkyle, trialkylammonium d'alkyle, amination réductrice, etc...voir par exemple Advanced Organic Chemistry, 3ème édition, 1985 , J. March , Willey Interscience*).*

La réduction du groupe azoïque conduit aux composés **e** et **h** conformes à l'invention.
L'étape de réduction est réalisée de manière classique, par exemple en effectuant une réaction d'hydrogénation par catalyse hétérogène en présence de Pd/C, Pd(II)/C, Ni/Ra, etc... ou encore en effectuant une réaction de réduction par un métal, par exemple par du zinc, fer, étain, etc. (voir Advanced Organic Chemistry, 3ème édition, J. March, 1985 , Willey Interscience et Reduction in organic Chemistry, M . Hudlicky, 1983 , Ellis Horwood Séries Chemical Science).

Selon un procédé nouveau, les dérivés 2,3-diamino-6,7-dihydro-1H,5H-pyrazolo[1,2-a]-pyrazol-1-one et 2,3-diamino-5,6,7,8-tétrahydro-1H,6H-pyridazino[1,2-a]-pyrazol-1-one conformes à la formule (I) sont obtenus selon la synthèse illustrée par le schéma F :

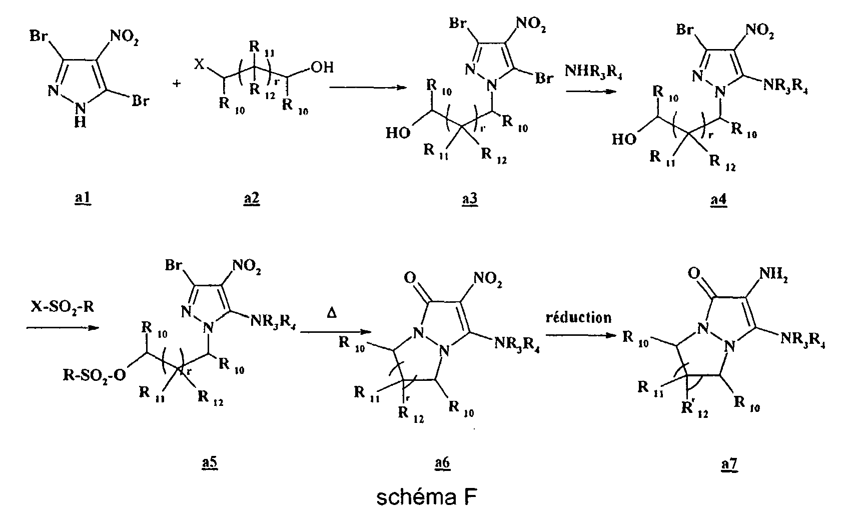

Selon ce procédé, on met en oeuvre les étapes suivantes :
a) étape 1 : on fait réagir un composé **a1** suivant : 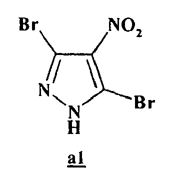 avec un composé **a2** : 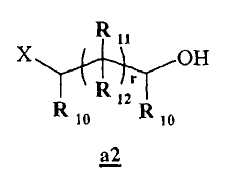 pour obtenir un composé **a3 :** 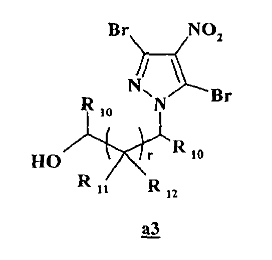 dans lesquelles :
   le radical R₁₀ représente un atome d'hydrogène, un carboxy ; un carboxamido ; un radical alkyle en C₁-C₄ éventuellement substitués par un ou plusieurs radicaux hydroxy, amino, (di)-alkylamino, alcoxy, carboxy, sulfonyle ;
   les radicaux R₁₁ et R₁₂ représentent indépendamment les uns des autres des atomes d'hydrogène, d'halogène ; des radicaux amino ; (di)alkyl(C₁-C₄)amino ; hydroxy; carboxy; carboxamido ; (C₁-C₂)alcoxy; radical alkyle en C₁-C₄ éventuellement substitués par un ou plusieurs radicaux hydroxy, amino, (di)-alkylamino, alcoxy, carboxy, sulfonyle ;
   X représente un atome d'halogène ou un alkyle sulfonate.
   r est un entier compris entre 1 et 3 ;
b) étape 2 : on fait réagir le composé **a3** avec une amine de formule NHR₃R₄ pour obtenir un composé **a4 :** 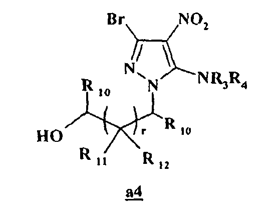
c) étape 3 : on fait réagir le composé **a4** avec au moins un halogénure d'alkylsulfonyle, d'arylsulfonyle ou de perfluoroalkylsulfonyle R-O₂S-X₁ (R représente un alkyle, un aryle ou un perfluoroalkyle, X₁ représente un halogène), dans un solvant de point d'ébullition compris entre 60°C et 190°C pour obtenir un composé **a5** : 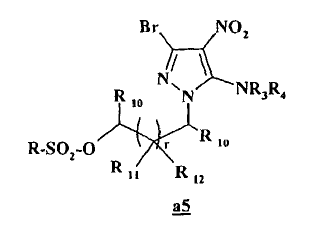
d) étape 4 : le composé **a5** résultant est ensuite chauffé dans un solvant de point d'ébullition compris entre 60°C et 190°C pour obtenir un composé **a6 :** 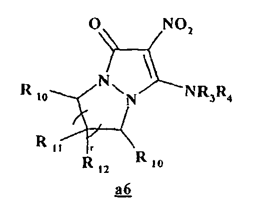
e) étape 5 : Le composé **a6** obtenu est réduit pour obtenir le composé **a7** de formule ci-dessous (III):

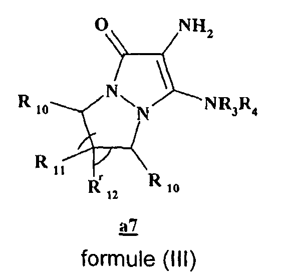

Plus particulièrement, suivant ce procédé, le 3,5-dibromo-4-nitropyrazole **a1,** obtenu par exemple selon la méthode décrite dans le DE 4234885, réagit avec le réactif **a2,** de préférence dans un solvant de point d'ébullition compris entre 60°C et 190°C. A titre d'exemple, on peut citer le pentanol, le diméthylformamide, la N-méthylpyrrolidine. Plus particulièrement, la réaction est effectuée en présence d'une base organique ou minérale, telle que par exemple le carbonate de sodium, l'hydroxyde de sodium, l'acétate de sodium, ou la triéthylamine. La température du milieu réactionnel est avantageusement maintenue entre 60°C et 160°C, de préférence entre 80°C et 120°C.

Le 1-hydroxyalkyl-3,5-dibromo-4-nitropyrazole **a3** est de préférence isolé par précipitation ou cristallisation après ajout de glace au milieu réactionnel.

Dans l'étape 2, le dérivé **a3** est mis en réaction avec une amine NHR₃R₄, de préférence dans un solvant de point d'ébullition compris entre 60°C et 190°C, tel que par exemple le butanol, le pentanol, le diméthylformamide. La température est plus particulièrement comprise entre 60°C et 160°C, de préférence entre 80°C et 120°C. Après consommation des réactifs, le composé 5-amino-4-nitro-3-bromo-1-hydroxyalkylpyrazole **a4** est isolé par précipitation ou cristallisation à l'aide d'eau.

Conformément à l'étape 3, le dérivé **a5** est obtenu par réaction de l'alcool **a4** et d'un halogénure d'alkylsulfonyle, d'arylsulfonyle ou de perfluoroalkylsulfonyle. La réaction a lieu de préférence dans un solvant aprotique tel que par exemple le tétrahydrofurane, le dioxane. La réaction a lieu avantageusement à une température comprise entre -20°C et 60°C, de préférence entre 0°C et 25°C. De plus, cette étape a lieu en présence d'une base organique ou minérale telle que par exemple le carbonate de potassium, la triéthylamine, la N-méthylmorpholine. Après disparition des réactifs, le composé **a5** est isolé par précipitation ou cristallisation dans l'eau.

Le sulfonate **a5** obtenu à l'issue de l'étape 3 est mis, dans l'étape 4, en solution ou en dispersion dans un solvant de point d'ébullition compris entre 60°C et 190°C, de préférence entre 90°C et 140°C. La température du milieu réactionnel est ensuite amenée entre 90°C et 140°C, de préférence entre 105°C et 125°C jusqu'à consommation totale du sulfonate **a5.** Après retour à la température ambiante, le composé perhydro-pyrazolo[1,2-a]pyrazol-1-one (r=1), perhydro-pyridazino[1,2-a]pyrazol-1-one (r=2) ou perhydro-diazépino[1,2-a]pyrazolone (r=3) **a6** cristallise et est isolé par les méthodes classiques de synthèse organique.

Le composé final **a7** conforme à l'invention est obtenu, lors d'une étape 5 par réduction du dérivé nitré **a6,** les méthodes de réduction utilisées étant par exemple une hydrogénation par catalyse hétérogène en présence de Pd/C, Pd(II)/C, Ni/Ra, etc... ou encore telles une réaction de réduction par un métal, par exemple par du zinc, fer, étain, etc, (voir Advanced Organic Chemistry, 3ème édition, J. March, 1985, Willey Interscience et Reduction in organic Chemistry, M. Hudlicky, 1983 ,Ellis Horwood Séries Chemical Science).

Les exemples qui suivent servent à illustrer l'invention.

### EXEMPLES

### Exemple 1 : synthèse du dichlorhydrate de 2,3-diamino-6,7-dihydro-1H,5H-pyrazolo[1,2-a]pyrazol-1-one 5

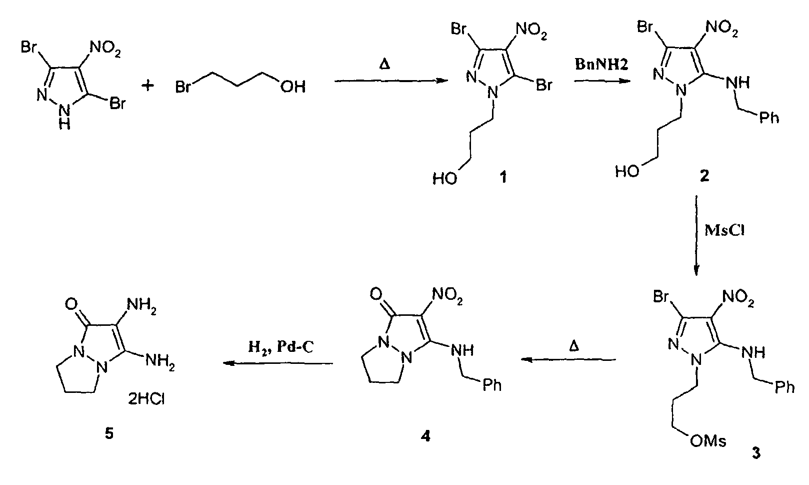

### -Etape 1 : synthèse du 3-(3,5-dibromo-4-nitro-1H-pyrazol-1-yl)propan-1-ol 1

Dans un tricol de 500 ml, on introduit 0.369 mole d'acétate de sodium à une solution de 0.184 mole de dibromonitropyrazole dans 250 ml de N-méthyl pyrrolidone et le milieu réactionnel est porté à 80°C.

A cette température, on ajoute au goutte à goutte 0.369 mole de 3-bromo propanol. Cette température est maintenue pendant 5 heures.
Après refroidissement à température ambiante, le milieu est versé sur de la glace sous agitation.
Le 3-(3,5-dibromo-4-nitro-1H-pyrazol-1-yl)propan-1-ol 1 précipite. Il est essoré, séché et obtenu avec un rendement de 75%.

La masse du composé attendu C₆H₇Br₂N₃O₃ est détectée en spectrométrie de masse.

Les analyses RMN (¹H 400 MHz et ¹³C 100,61 MHz DMSO d₆) sont conformes à la structure attendue.

### -Etape 2 : synthèse du 3-[5-(benzylamino)-3-bromo-4-nitro-1H-pyrazol-1-yl]propan-1-ol 2

Dans un tricol de 500 ml contenant 150 ml d'éthanol, on disperse 0.135 mole de 3-(3,5-dibromo-4-nitro-1H-pyrazol-1-yl)propan-1-ol **1**, chauffe à 60°C, puis additionne 0.825 mole de benzylamine en 30 minutes.

Après 6 heures à 60°C, le milieu réactionnel est refroidi à température ambiante.
Le 3-[5-(benzylamino)-3-bromo-4-nitro-1H-pyrazol-1-yl]propan-1-ol **2** est précipité en versant le milieu réactionnel sur 1 litre de glace sous agitation. Après essorage et séchage sous vide en présence de P₂O₅, le composé 2 est isolé avec un rendement de 90%.

Les analyses RMN (¹H 400 MHz et ¹³C 100,61 MHz DMSO d₆) sont conformes à la structure attendue.

**Analyse élémentaire :**

| | | | | | |
|---|---|---|---|---|---|
| théorique : | C43.96 | H4.26 | N15.77 | O13.51 | Br22.50 |
| mesuré : | C44.09 | H4.22 | N15.44 | O14.37 | Br21.50 |

### -Etape 3 : synthèse du 3-[5-(benzylamino)-3-bromo-4-nitro-1H-pyrazol-1-yl]propyl méthanesulfonate 3

Dans un tricol de 500 ml contenant 200 ml de THF, on introduit, sous agitation, 0.126 mole de 3-[5-(benzylamino)-3-bromo-4-nitro-1H-pyrazol-1-yl]propan-l-ol **2** et 15.82 ml de triéthylamine. Le mélange obtenu est ensuite refroidi à 5°C et 0.126 mole de chlorure de mésyle sont coulés en 45 minutes.
Le milieu réactionnel est maintenu à cette température pendant 2 heures, puis le 3-[5-(benzylamino)-3-bromo-4-nitro-1H-pyrazol-1-yl]propylmethanesulfonate **3** est précipité en versant le milieu réactionnel sur 800 ml de la glace.

Après filtration, le solide est lavé abondamment à l'eau et à l'éther diisopropylique. Le séchage est réalisé sous vide en présence de P₂O₅. Le rendement de cette étape est de 94%

La masse du composé attendu C₁₄H₁₇BrN₄O₅S est détectée en spectrométrie de masse.

Les analyses RMN (¹H 400 MHz et ¹³C 100,61 MHz DMSO d₆) sont conformes à la structure attendue.

**Analyse élémentaire :**

| | | | | | | |
|---|---|---|---|---|---|---|
| Théorie : | C38.81 | H3.96 | N12.93 | O18.46 | S7.40 | Br18.44 |
| Mesuré : | C39.03 | H3.91 | N12.83 | O18.52 | S7.29 | Br18.26 |

### -Etape 4 : synthèse de la 3-(benzylamino)-2-nitro-6,7-dihydro-1H,5H-pyrazolo[1,2-a]pyrazol-1- one 4

Dans un tricol de 500 ml contenant 300 ml de pentanol, on disperse sous agitation 0.1 mole de 3-[5-(benzylamino)-3-bromo-4-nitro-1H-pyrazol-1-yl]propyl méthanesulfonate **3** et porte le milieu réactionnel à 130°C pendant 2 heures.

Après refroidissement à température ambiante, le solide formé est essoré sur fritté, lavé à l'éther diisopropylique et séché sous vide en présence de P₂O₅, Le 3-(benzylamino)-2-nitro-6,7-dihydro-1H,5H-pyrazolo[1,2-a]pyrazol-1- one 4 est obtenu avec un rendement de 86%.
Les analyses RMN (¹H 400 MHz et ¹³C 100,61 MHz DMSO d₆) sont conformes à la structure attendue.
La masse du composé attendu C₆H₁₁N₄O est détectée en spectrométrie de masse.

**Analyse élémentaire :**

| | | | | |
|---|---|---|---|---|
| Théorie : | C56.72 | H5.49 | N20.36 | O17.44 |
| Mesuré : | C56.68 | H5.13 | N20.38 | O17.69 |

### -Etape 5 : synthèse du dichlorhydrate de 2,3-diamino-6,7-dihydro-1H,5H-pyrazolo[1,2-a]pyrazol-1-one 5

Dans un autoclave de 1 litre contenant 800 ml d'éthanol, on introduit 20 g de 3-(benzylamino)-2-nitro-6,7-dihydro-1H,5H-pyrazolo[1,2-a]pyrazol-1-one **4** et 4g de palladium sur charbon à 5%. La réduction est ensuite réalisée sous une pression d'hydrogène de 8 Bars et à une température comprise entre 50°C et 100°C ( agitation comprise entre 1000 et 2500 tr/min)

Au bout de 4 heures de réaction, il n'a plus consommation d'hydrogène et le milieu est refroidi à 20°C.

Le catalyseur est éliminé sous azote par filtration, puis de l'éthanol chlorhydrique est ajouté au filtrat. Le produit cristallisé est essoré, lavé à l'éther diisopropylique, puis séché sous vide en présence de P₂O₅. Le dichlorhydrate de 2,3-diamino-6,7-dihydro-1H,5H-pyrazolo[1,2-a]pyrazol-1-one **5** est obtenu avec un rendement de 89%.
La masse du composé attendu est détectée en spectrométrie de masse.
Les analyses RMN (¹H 400 MHz et ¹³C 100,61 MHz DMSO d₆) sont conformes à la structure attendue.

**Analyse élémentaire :**

| | | | | | |
|---|---|---|---|---|---|
| Théorique : | C31.73 | H5.33 | N24.67 | O7.07 | C131.22 |
| Mesuré : | C31.45 | H5.20 | N24.62 | O7.24 | C130.86 |

### Exemple 2 : synthèse du dichlorhydrate de 2-amino-3-(éthylamino)-6,7-dihydro-1H,5H-pyrazolo[1,2-a]pyrazol-1-one 9

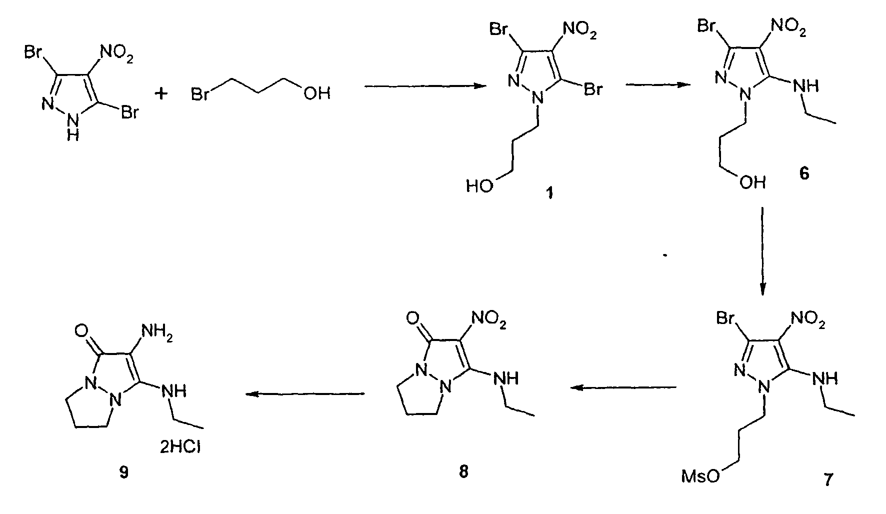

### -Etape 2 synthèse du 3-[3-bromo-5-(éthylamino)-4-nitro-1H-pyrazol-1-yl]propan-1-ol 6

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75°C puis 93 mmoles d'éthylamine sont coulées au goutte à goutte et l'agitation est maintenue quatre heures.

Après refroidissement à température ambiante, le milieu est versé sur de la glace et le 3-[3-bromo-5-(éthylamino)-4-nitro-1 H-pyrazol-1-yl]propan-1-ol 6 précipite.

Le solide jaune est essoré, puis lavé abondamment à l'eau et à l'éther diisopropylique. Le séchage est réalisé sous vide en présence de P₂O₅. La masse récupérée est de 3.6 g.

Les analyses RMN (¹H 400 MHz et ¹³C 100,61 MHz DMSO d₆) sont conformes à la structure attendue.

La masse du composé attendu C₈H₁₃BrN₄O₃ est détectée en spectrométrie de masse.

### -Etape 3 : synthèse du 3-[5-(éthylamino)-3-bromo-4-nitro-1H-pyrazol-1-yl]propyl methanesulfonate 7

Dans un tricol de 100 ml contenant 30 ml de THF, sous agitation, on introduit 11.2 mmoles de 3-[3-bromo-5-(éthylamino)-4-nitro-1H-pyrazol-1-yl]propan-1-ol **6** et 1.6 ml de triéthylamine. Le mélange homogène orange obtenu est refroidi à 0°C et 1.44 ml de chlorure de mésyle sont coulés en 20 minutes.

Le milieu réactionnel est maintenu à cette température pendant 2 heures puis le 3-[5-(éthylamino)-3-bromo-4-nitro-1H-pyrazol-1-yl]propylméthanesulfonate **7** est précipité en versant le milieu réactionnel sur 500 ml de glace.

Le solide jaune est essoré, puis lavé abondamment à l'eau et à l'éther diisopropylique ; le séchage est réalisé sous vide en présence de P₂O₅. La masse récupérée est de 3.1 g.

Les analyses RMN (¹H 400 MHz et ¹³C 100,61 MHz DMSO d₆) sont conformes à la structure attendue.

La masse du composé attendu C₉H₁₅BrN₄O₅S est détectée en spectrométrie de masse.

### -Etape 4 : synthèse de la 3-(éthylamino)-2-nitro-6,7-dihydro-1H,5H-pyrazolo[1,2-a]pyrazol-1-one 8

Dans un tricol de 50 ml contenant 20 ml de pentanol, on disperse sous agitation 8 mmoles de 3-[5-(éthylamino)-3-bromo-4-nitro-1H-pyrazol-1-yl]propyl méthanesulfonate **7**, et porte le milieu réactionnel à 130°C pendant 2 heures.
Après refroidissement à température ambiante, le solide formé est essoré, puis lavé à l'éther diisopropylique.

Après séchage sous vide en présence de P₂O₅, on obtient 1.46 g de 3-(éthylamino)-2-nitro-6,7-dihydro-1H,5H-pyrazolo[1,2-a]pyrazol-1 one **8**.

Les analyses RMN (¹H 400 MHz et ¹³C 100,61 MHz DMSO d₆) sont conformes à la structure attendue.

La masse du composé attendu est détectée en spectrométrie de masse.

### -Etape 5 : synthèse du dichlorhydrate de 2-amino-3-(éthylamino)-6,7-dihydro-1H,5H-pyrazolo[1,2-a]pyrazol-1-one 9

Dans un autoclave de 300 ml contenant 200 ml d'éthanol, on introduit 1.45 g de 3-(éthylamino)-2-nitro-6,7-dihydro-1H,5H-pyrazolo[1,2-a]pyrazol-1-one **8** et 300 mg de palladium sur charbon à 5%. La réduction est réalisée sous une pression d'hydrogène de 8 Bars à une température de 60°C (agitation de 1700 tr/min).
Au bout de 2 heures de réaction, il n'y a plus consommation d'hydrogène et le milieu est refroidi à 20°C.

Le catalyseur est éliminé par filtration sous azote et le filtrat est dilué avec 100 ml d'éther isopropylique chlorhydrique.

La solution jaune pale est évaporée à sec puis le solide est repris avec un mélange éthanol / éther isopropylique. Le dichlorhydrate de 2-amino-3-(éthylamino)-6,7-dihydro-1H,5H-pyrazolo[1,2-a]pyrazol-1-one **9** précipite; il est essoré et après séchage sous vide en présence de P₂O₅, on récupère 1.18 g de dichlorhydrate de 2-amino-3-(éthylamino)-6,7-dihydro-1H,5H-pyrazolo[1,2-a]pyrazol-1-one **9**.
Les analyses RMN (¹H 400 MHz et ¹³C 100,61 MHz DMSO d₆) sont conformes à la structure attendue.
La masse du composé attendu C₈H₁₄N₄O est détectée en spectrométrie de masse.

### Exemple 3 : dichlorhydrate de 2-amino-3-(isopropylamino)-6,7-dihydro-1H,5H-pyrazolo[1,2-a]pyrazol-1-one 13

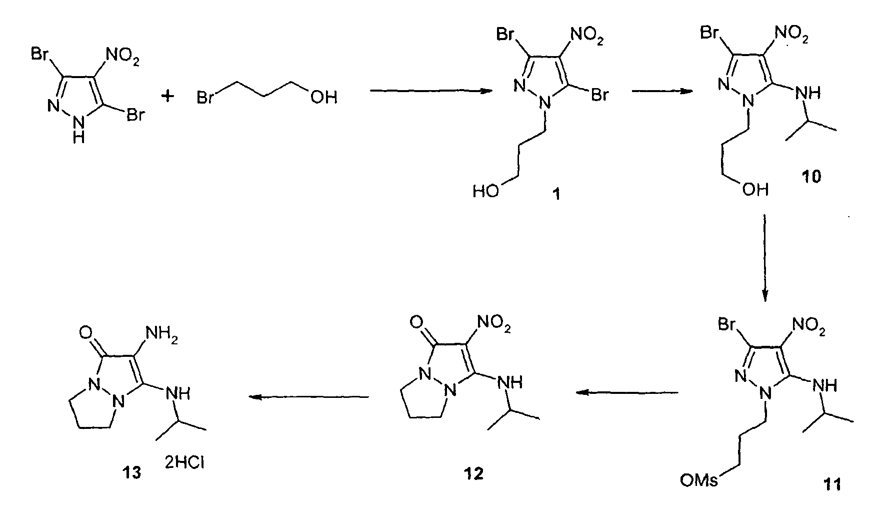

### -Etape 2 : 3-[3-bromo-5-(isopropylamino)-4-nitro-1H-pyrazol-1-yl]propan-1-ol 10

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75°C, puis 93 mmoles d'isopropylamine sont coulées au goutte à goutte tout en maintenant l'agitation quatre heures.

Après refroidissement à température ambiante, le milieu est versé sur de la glace, puis neutralisé à l'acide chlorhydrique. On extrait le 3-[3-bromo-5-(isopropylamino)-4-nitro-1 H-pyrazol-1-yl]propan-1-ol **10** au dichlorométhane.

Après séchage de la phase organique sur sulfate de sodium et élimination du solvant par évaporation sous vide, on obtient 4.37 g de 3-[3-bromo-5-(isopropylamino)-4-nitro-1 H-pyrazol-1-yl]propan-1-ol **10**.

Les analyses RMN (¹H 400 MHz et ¹³C 100,61 MHz DMSO d₆) sont conformes à la structure attendue.

La masse du composé attendu C₉H₁₅BrN₄O₃ est détectée en spectrométrie de masse.

### -Etape 3 : synthèse du 3-[5-(isopropylamino)-3-bromo-4-nitro-1H-pyrazol-1-yl]propyl méthanesulfonate 11

Dans un tricol de 50 ml contenant 20 ml de THF, on introduit, sous agitation, 13.7 mmoles de 3-[3-bromo-5-(isopropylamino)-4-nitro-1H-pyrazol-1-yl]propan-1-ol **1**0 et 1.94 ml de triéthylamine. Le mélange homogène orange ainsi obtenu est refroidi à 0°C et 1.76 ml de chlorure de mésyle sont coulés en 20 minutes.

Le milieu réactionnel est maintenu à cette température pendant 2 heures, puis le 3-[5-(éthylamino)-3-bromo-4-nitro-1H-pyrazol-1-yl]propylméthanesulfonate **11** est précipité en versant le milieu réactionnel sur 500 ml de glace.

Le solide jaune est essoré, puis lavé abondamment à l'eau et à l'éther de pétrole, le séchage est réalisé sous vide en présence de P₂O₅. La masse récupérée est de 4.2 g.

Les analyses RMN (¹H 400 MHz et ¹³C 100,61 MHz DMSO d₆) sont conformes à la structure attendue.

La masse du composé attendu est détectée en spectrométrie de masse..

### -Etape 4 : synthèse de la 3-(isopropylamino)-2-nitro-6,7-dihydro-1H,5H-pyrazolo[1,2-a]pyrazol-1-one 12

Dans un tricol de 50 ml, on disperse, sous agitation, 10 mmoles de 3-[5-(isopropylamino)-3-bromo-4-nitro-1H-pyrazol-1-yl]propylméthanesulfonate **11** dans 20 ml de pentanol et chauffe à 130°C pendant 2 heures.

Après refroidissement à température ambiante, le solide obtenu est essoré sur fritté, et lavé à l'éther diisopropylique.

Après séchage sous vide en présence de P₂O₅, 1,71 g de 3-(isopropylamino)-2-nitro-6,7-dihydro-1H,5H-pyrazolo[1,2-a]pyrazol-1one **12** sont obtenus

Les analyses RMN (¹H 400 MHz et ¹³C 100,61 MHz DMSO d₆) sont conformes à la structure attendue.

La masse du composé attendu C₉H₁₄N₄O₃ est détectée en spectrométrie de masse.

### -Etape 5 : synthèse du dichlorhydrate de 2-amino-3-(isopropylamino)-6,7-dihydro-1H,5H-pyrazolo[1,2-a]pyrazol-1-one 13

Dans un autoclave de 300 ml contenant 200 ml d'éthanol, on introduit 1.70 g de 3-(isopropylaminoamino)-2-nitro-6,7-dihydro-1H,5H-pyrazolo[1,2-a]pyrazol-1-one **12** et 300 mg de palladium sur charbon à 5%. La réduction est réalisée sous une température de 60°C et sous une pression d'hydrogène de 6 Bars (agitation de 2000 tr/min).

Au bout de 2 heures de réaction, il n'a plus consommation d'hydrogène et le milieu est refroidi à 20°C.
Le catalyseur est éliminé par filtration sous azote après refroidissement à température ambiante et de l'éther isopropylique chlorhydrique est additionné.

La solution jaune pale est évaporée à sec, puis le solide est repris avec 50 ml d'éther diisopropylique saturé en acide chlorhydrique, le précipité est récupéré par essorage. Après séchage sous vide en présence de P₂O₅, 1,5 g de dichlorhydrate de 2-amino-3-(isopropylamino)-6,7-dihydro-1H,5H-pyrazolo[1,2-a]pyrazol-1-one **13** sont isolés.

Les analyses RMN (¹H 400 MHz et ¹³C 100,61 MHz DMSO d₆) sont conformes à la structure attendue.

La masse du composé attendu C₉H₁₆N₄O est détectée en spectrométrie de masse.

### Exemple 4 : dichlorhydrate de 2-amino-3-(pyrrolidin-1-yl)-6,7-dihydro-1H,5H-pyrazolo[1,2-a]pyrazol-1-one 17

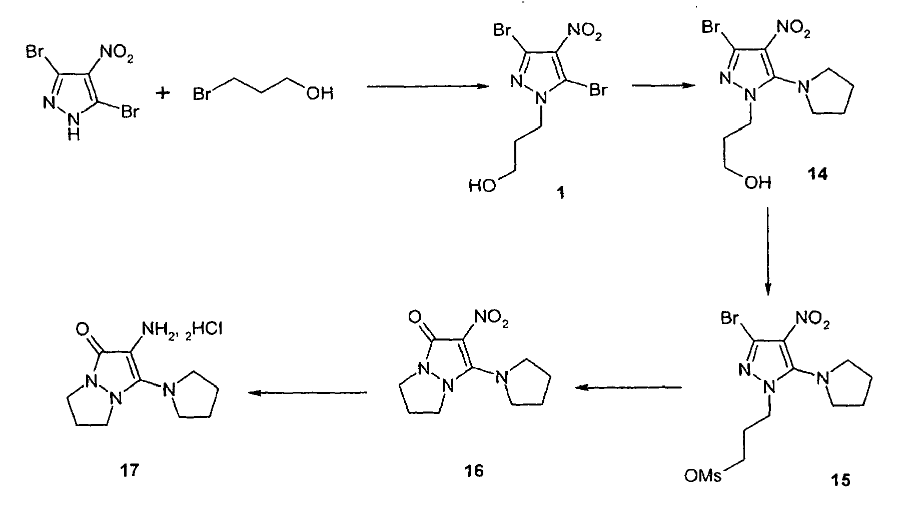

### -Etape 2: 3-(3-bromo-4-nitro-5-(pyrrolidin-1-yl)-1H-pyrazol-1-yl)propan-1-ol 14

Dans un tricol, sous agitation, on introduit 15 mmoles de 3-(3,5-dibromo-4-nitro-1H-pyrazol-1-yl)propan-1-ol dans 20 ml d'isopropanol. Le milieu homogène est chauffé à 75°C puis 90 mmoles de pyrrolidine sont coulées au goutte à goutte et l'agitation est maintenue deux heures.

Après refroidissement à température ambiante, le milieu est versé sur de la glace et neutralisé à l'acide chlorhydrique. On extrait le 3-(3-bromo-4-nitro-5-(pyrrolidin-1-yl)-1H-pyrazol-1-yl)propan-1-ol **14** par du dichlorométhane.

Après séchage de la phase organique sur sulfate de sodium et distillation du solvant par évaporation sous vide, on obtient 4.8 g de 3-(3-bromo-4-nitro-5-(pyrrolidin-1-yl)-1H-pyrazol-1-yl)propan-1-ol **14**.

Les analyses RMN (¹H 400 MHz et ¹³C 100,61 MHz DMSO d₆) sont conformes à la structure attendue.

La masse du composé attendu C₁₀H₁₇BrN₄O est détectée en spectrométrie de masse.

### -Etape 3 : synthèse du 3-(3-bromo-4-nitro-5-(pyrrolidin-1-yl)-1H-pyrazol-1-yl)propylméthanesulfonate 15

Dans un tricol de 100 ml contenant 50 ml de THF, on introduit, sous agitation, 30 mmoles 3-(3-bromo-4-nitro-5-(pyrrolidin-1-yl)-1H-pyrazol-1-yl)propan-1-ol **14** et 4.25 ml de triéthylamine. Le mélange homogène orange obtenu est refroidi à 0°C et 2.32 ml de chlorure de mésyle sont coulés en 20 minutes.

Le milieu réactionnel est maintenu à cette température pendant 2 heures puis le 3-(3-bromo-4-nitro-5-(pyrrolidin-1-yl)-1H-pyrazol-1-yl)propylméthanesulfonate **15** est précipité en versant le milieu réactionnel sur de la glace.
Le solide est essoré, puis séché sous vide en présence de P₂O₅. La masse récupérée est de 9.3 g.

Les analyses RMN (¹H 400 MHz et ¹³C 100,61 MHz DMSO d₆) sont conformes à la structure attendue.

La masse du composé attendu C₁₁H₁₉BrN₄O₃S est détectée en spectrométrie de masse.

### -Etape 4 : synthèse 2-nitro-3-(pyrrolidin-1-yl)-6,7-dihydro-1H_{.}5H-pyrazolo[1,2-a]pyrazol-1-one 16

Dans un tricol de 250 ml, on introduit, sous agitation, 22.5 mmoles de 3-(3-bromo-4-nitro-5-(pyrrolidin-1-yl)-1H-pyrazol-1-yl)propylméthanesulfonate **15** dans 100 ml de pentanol. Le milieu ainsi obtenu est porté à 130°C pendant 2 heures.

Après refroidissement à température ambiante, le 2-nitro-3-(pyrrolidin-1-yl)-6,7-dihydro-1H,5H-pyrazolo[1,2-a]pyrazol-1-one **16** est extrait au dichlorométhane.

Après séchage de la phase organique sur sulfate de sodium et distillation du solvant sous vide, on obtient 1.2 g de 2-nitro-3-pyrrolidin-1-yl-6,7-dihydro-1H,5H-pyrazolo[1,2-a]pyrazol-1-one **16**

Les analyses RMN (¹H 400 MHz et ¹³C 100,61 MHz DMSO d₆) sont conformes à la structure attendue..

La masse du composé attendu C₁₀H₁₄N₄O₃ est détectée en spectrométrie de masse.

### -Etape 5 : synthèse du dichlorhydrate de 2-amino-3-(pyrrolidin-1-yl)-6,7-dihydro-1H,5H-pyrazolo[1,2-a]pyrazol-1-one 17

Dans un autoclave de 300 ml contenant 200 ml d'éthanol, on introduit 1.1 g de 2-nitro-3-(pyrrolidin-1-yl)-6,7-dihydro-1H,5H-pyrazolo[1,2-a]pyrazol-1-one 16 et 300 mg de palladium sur charbon à 5%. La réduction est réalisée sous une agitation de 2000 tr/min, sous une température de 60°C et sous une pression d'hydrogène de 6 Bars.

Au bout de 2 heures de réaction, il n'a plus consommation d'hydrogène et le milieu est refroidit à 20°C.

Le catalyseur est éliminé par filtration sous azote après refroidissement à température ambiante et de l'éther isopropylique chlorhydrique est additionné.

La solution jaune pale est évaporée à sec, puis le solide est repris avec 50 ml d'éther diisopropylique saturé en acide chlorhydrique, le précipité est récupéré par essorage. Après séchage sous vide en présence de P₂O₅, on obtient 1.5 g de dichlorhydrate de 2-amino-3-(pyrrolidin-1-yl)-6,7-dihydro-1H,5H-pyrazolo[1,2-a]pyrazol-1-one 5 **17.**

Les analyses RMN (¹H 400 MHz et ¹³C 100,61 MHz DMSO d₆) sont conformes à la structure attendue.

La masse du composé attendu C₁₀H₁₆N₄O est détectée en spectrométrie de masse.

### Exemple 5. Synthèse du diméthanesulfonate de 2,3-diamino-6,7-dihydro-1H,5H-pyrazolo[1,2-a]pyrazol-1-one

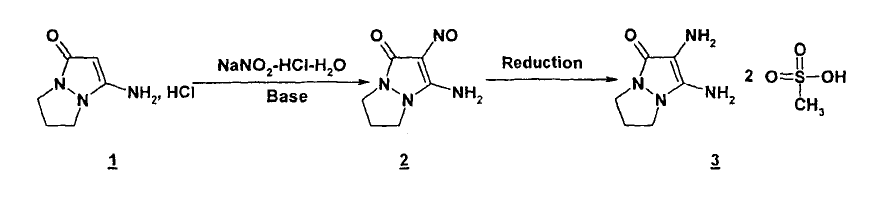

### Synthèse du 3-amino-2-nitroso-6,7-dihydro-1H,SH-pyrazolo[1,2-a]pyrazol-1-one: 2

Dans un tricol de 500 ml, on dissout, sous agitation, à température ambiante, 43g (0.245 mole) de chlorhydrate de 3-amino-6,7-dihydro-1H,5H-pyrazolo[1,2-a]pyrazol-1-one dans un mélange de 180 ml d'eau et 35 ml d'acide chlorhydrique à 35%.

On refroidit à 0°C et ajoute goutte à goutte, en 30 minutes, une solution de 17.3 g de nitrite de sodium (0.25 mole) dans 20 ml d'eau. La température du milieu réactionnel est maintenue entre 0 et +5°C pendant toute la durée de l'addition et pendant une heure après la fin de l'addition.

Le milieu réactionnel est amené à pH 8 par addition de soude, sous agitation, tout en maintenant la température entre 0 et 5°C. La 3-amino-2-nitroso-6,7-dihydro-1H,5H-pyrazolo[1,2-a]pyrazol-1-one 2 précipite sous la forme d'un solide orangé rouge qui est filtré sur verre fritté n°4, empâté dans le minimum de 2-propanol, lavé à l'éther diisopropylique et séché sous vide en présence de pentaoxyde de phosphore. On obtient ainsi 35 g de produit rouge orangé (rendement : 85%).

Les spectres de RMN (1 H 400 MHz et 13C 100,61 MHz DMSO d6) et de masse sont conformes à la structure attendue 2.

### Synthèse du diméthanesulfonate de 2,3-diamino-6,7-dihydro-1H,5H-pyrazolo[1,2-a]pyrazol-1-one : 3

Dans un autoclave de 1 litre, on introduit 33.6 g (0.2mole) de 3-amino-2-nitroso-6,7-dihydro-1 H,5H-pyrazolo[1,2-a]pyrazol-1-one **2,** 500 ml d'éthanol et 6 g de palladium sur charbon à 5% contenant 50% d'eau.

Le milieu est purgé 3 fois à l'azote puis 3 fois à l'hydrogène et la température du mélange est portée à 40°C.
La réduction est réalisée en deux heures sous une pression de 8 bars. Cette réduction est exothermique et la température atteint d'elle-même 70°C.
On laisse la température redescendre à 50°C puis le catalyseur est filtré sur un filtre presse sous un courant d'azote.
Le filtrat est coulé dans un mélange de 50 ml d'éthanol et 40 ml d'acide méthane sulfonique, en refroidissant à 0°C. Le diméthanesulfonate de 2,3-diamino-6,7-dihydro-1 H,SH-pyrazolo[1,2-a]pyrazol-1-one **3** cristallise sous la forme d'un solide jaune pâle qui est essoré sur verre fritté n°4, lavé à l'éther diisopropylique puis à l'éther de pétrole et enfin séché sous vide en présence de pentaoxyde de phosphore. On obtient ainsi 43g de solide jaune pâle (rendement : 65%).
Les spectres de RMN (¹H 400 MHz et ¹³C 100,61 MHz DMSO d₆) et de masse sont conformes à la structure attendue **3**.

**Analyse élémentaire :**

| | | | | | |
|---|---|---|---|---|---|
| Théorie : | C27.74 | H5.23 | N16.17 | O32.33 | S18.51 |
| Mesuré : | C27.16 | H5.22 | N15.63 | O32.81 | S18.64 |

### Exemple 6 : synthèse du chlorhydrate de 2,3-diamino-5,6,7,8-tetrahydro-1H-pyrazolo[1,2-a]pyridazin-1-one.

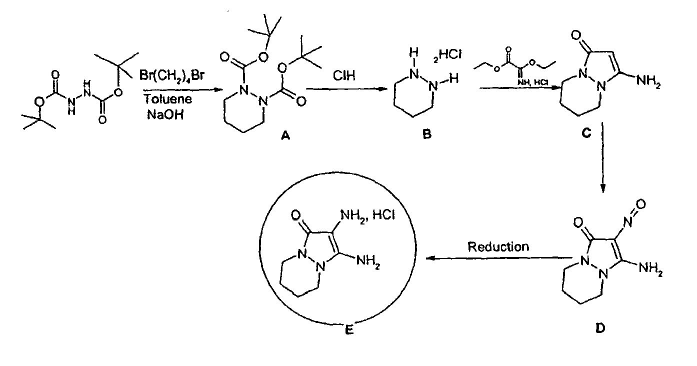

### Synthèse du di-tert-butyl tetrahydropyridazine-1,2-dicarboxylate : A

Dans un tricol de 250 ml équipé d'un réfrigérant, d'un thermomètre et d'une ampoule de coulée, on introduit sous agitation mécanique 50 ml de toluène, 5 g (21.5 mmoles) de N,N'-di-tert-butoxycarbonylhydrazide, 680 mg de bromure de tétraéthylammonium et 25 ml de soude à 50%.
Le milieu hétérogène est chauffé à 100°C puis on ajoute goutte à goutte en 15 minutes le 1,4-dibromobutane.
Le milieu réactionnel est chauffé à 100°C pendant 3 jours. Après refroidissement, on ajoute 100 ml d'acétate d'éthyle et on transfère dans une ampoule à décanter. La phase organique est lavée avec 4 fois 70 ml de solution aqueuse saturée en carbonate de sodium, puis avec 4 x 70 ml d'eau et enfin avec 4 x 70ml d'eau salée. La phase organique est séchée sur sulfate de sodium et le solvant est évaporé sous vide. On obtient ainsi une huile incolore qui cristallise en un solide blanc.
On récupère une masse de 6.1 g (rendement : 99%).
Les spectres RMN (¹H 400 MHz et ¹³C 100,61 MHz DMSO d₆) et de masse sont conformes à la structure attendue **A**.

### Synthèse du dichlorhydrate d'hexahydropyridazine : B

Dans un tricol de 100 ml équipé d'un réfrigérant et d'un thermomètre, on introduit sous agitation mécanique 5.9 g du composé **A** dans 50 ml de mélange 3/1 de dioxanne et d'acide chlorhydrique à 35%.
La solution incolore obtenue est agitée à température ambiante pendant 3 heures puis le milieu réactionnel est dilué par de l'éther diisopropylique. Les solvants sont évaporés sous vide. Le résidu pâteux obtenu est repris par un mélange éther/éthanol. Après filtration du solide et séchage sous vide, on obtient 1.39 g de solide blanc.

Les spectres de RMN (¹H 400 MHz et ¹³C 100,61 MHz DMSO d₆) et de masse sont conformes à la structure attendue **B.**

### Synthèse du 3-amino-5,6,7,8-tetrahydro-1H-pyrazolo[1,2-a]pyridazin-1-one : C

Dans un tricol de 25 ml équipé d'un réfrigérant et d'un thermomètre, on introduit sous agitation mécanique 7.5 ml d'éthanol, 1,5 ml de triéthylamine et 0.73 ml d'acide 3-amino-3-ethoxyacrylique. On ajoute ensuite 500 mg de dichlorhydrate d'hexahydropyridazine (composé **B**) et on agite pendant 3 heures à température ambiante.
On filtre l'insoluble et distille le solvant sous vide. Le solide est repris par le minimum d'eau, filtré et séché sous vide. On obtient ainsi 0.9 g de poudre légèrement jaune.

Les spectres de RMN (¹H 400 MHz et ¹³C 100,61 MHz DMSO d₆) et de masse sont conformes à la structure attendue **C**.

### Synthèse du 3-amino-2-nitroso-5,6,7,8-tetrahydro-1H-pyrazolo[1,2-a]pyridazin-1-one D

Dans un tricol de 50 ml équipé d'un réfrigérant et d'un thermomètre, on introduit sous agitation mécanique 20 ml d'acide chlorhydrique à 35% et 1g de 3-amino-5,6,7,8-tetrahydro-1H-pyrazolo[1,2-a]pyridazin-1-one (composé **C**).
On refroidit à 0°C et on coule une solution de 675 mg nitrite de sodium dans 5 ml d'eau en maintenant cette température. La couleur du mélange réactionnel vire du jaune à l'orange et un précipité commence à se former.
En 30 minutes, la réaction est terminée et le solide orange est filtré sur verre fritté n°4, lavé à l'eau puis séché sous vide. Le rendement est de 78.3%.
Les spectres de RMN (¹H 400 MHz et ¹³C 100,61 MHz DMSO d₆) masse sont conformes à la structure attendue **D.**

### Synthèse du chlorhydrate de 2,3-diamino-5,6,7,8-tetrahydro-1H-pyrazolo[1,2-a]pyridazin-1-one : E

Dans un autoclave de 300 ml contenant 250 ml d'éthanol, on introduit 1.3 g de 3-amino-2-nitroso-5,6,7,8-tetrahydro-1H-pyrazolo[1,2-a]pyridazin-1-one (composé **D**) et 250 mg de palladium sur charbon à 5%. La réduction est réalisée sous une agitation de 2000 tr/min, à une température de 60°C et sous une pression d'hydrogène de 6 bars.
Au bout de 2 heures de réaction, il n'y a plus de consommation d'hydrogène et le milieu est refroidi à 20°C.
Le catalyseur est éliminé par filtration sous azote après refroidissement à température ambiante et la solution est versée sur 75 ml de dioxanne chlorhydrique.
La solution ainsi obtenue est évaporée jusqu'à obtention d'une poudre légèrement jaune que l'on reprend dans de l'éther diisopropylique.

Le solide est récupéré par filtration. Après séchage sous vide en présence de pentaoxyde de phosphore, on obtient 1.1 g de dichlorhydrate de 2,3-diamino-5,6,7,8-tetrahydro-1H-pyrazolo[1,2-a]pyridazin-1-one.
Les spectres de RMN (¹H 400 MHz et ¹³C 100,61 MHz DMSO d₆) et de masse sont conformes à la structure attendue **E.**

### EXEMPLES DE TEINTURE

### Exemples 1 à 3 : Teinture en milieu acide à partir du 2,3-diamino-6,7-dihydro-1H,5H-pyrazolo[1,2-a]pyrazol-1-one

Les compositions tinctoriales suivantes sont préparées :

| **Exemple** | **1** | **2** | **3** |
|---|---|---|---|
| Dichlorhydrate de 2,3-diamino-6,7-dihydro-1H,5H-pyrazolo[1,2-a]pyrazol-1-one | 10⁻³ mole | 10⁻³ mole | 10⁻³ mole |
| 5-Amino-2-méthyl-phenol | 10⁻³ mole | | |
| 2-(2,4-Diamino-phenoxy)-ethanol,chlorhydrate | | 10⁻³ mole | |
| 3-Amino-2-chloro-6-méthyl-phenol,chlorhydrate | | | 10⁻³ mole |
| Support de teinture (2) | (*) | (*) | (*) |
| Eau déminéralisée q.s.p. | 100g | 100g | 100g |

| | | | |
|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous:

| **Exemple** | **1** | **2** | **3** |
|---|---|---|---|
| **Nuance observée** | orangé chromatique | rouge chromatique intense | orangé chromatique |

### Exemples 4 à 6 : Teinture en milieu alcalin à partir du 2,3-diamino-6,7-dihydro-1H,5H-pyrazolo[1,2-a]pyrazol-1-one

Les compositions tinctoriales suivantes sont préparées :

| **Exemple** | **4** | **5** | **6** |
|---|---|---|---|
| Dichlorhydrate de 2,3-diamino-6,7-dihydro-1H,5H-pyrazolo[1,2-a]pyrazol-1-one | 10⁻³ mole | 10⁻³ mole | 10⁻³ mole |
| 5-Amino-2-méthyl-phenol | 10⁻³ mole | | |
| 2-(2,4-Diamino-phenoxy)-ethanol,chlorhydrate | | 10⁻³ mole | |
| 3-Amino-2-chloro-6-méthyl-phenol,chlorhydrate | | | 10⁻³ mole |
| Support de teinture (1) | (*) | (*) | (*) |
| Eau déminéralisée q.s.p. | 100g | 100g | 100g |

| | | | |
|---|---|---|---|
| (*) : support de teinture (1) pH 9,5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous:

| **Exemple** | **4** | **5** | **6** |
|---|---|---|---|
| **Nuance observée** | orangé chromatique | rouge chromatique | orangé chromatique |

## Revendications

1. Composition tinctoriale des fibres kératiniques comprenant, dans un milieu de teinture approprié, à titre de base d'oxydation, au moins un dérivé de la diamino-N,N-dihydro-pyrazolone de formule (I) ou l'un de ses sels d'addition ou solvates : 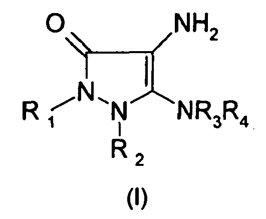 dans laquelle :
R₁ et R₂ forment avec les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R₃ et R₄, identiques ou différents, représentent :
- un radical alkyle en C₁-C₆ linéaire ou ramifié éventuellement substitué par un ou plusieurs radicaux choisi dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un groupe (C₁-C₄)alkyle, un hydroxy, un alcoxy en C₁-C₂, un amino, un (di)alkyl(C₁-C₂)amino;
- un radical aryle éventuellement substitué par un ou plusieurs (C₁-C₄)alkyle, hydroxy, alcoxy en C₁-C₂, amino, (di)alkyl(C₁-C₂)amino ;
- un radical hétéroaryle à 5 ou 6 chaînons, éventuellement substitué par un ou plusieurs radicaux choisis parmi (C₁-C₄)alkyle, (C₁-C₂)alcoxy;
R₃ et R₄ peuvent représenter également un atome d'hydrogène ;
R₅, R₆ et R₇, identiques ou différents, représentent un atome d'hydrogène ; un radical alkyle linéaire ou ramifié en C₁-C₄ éventuellement substitué par un ou plusieurs radicaux choisis dans le groupe constitué par un hydroxy, un alcoxy en C₁-C₂, un carboxamido CONR₈R₉, un sulfonyle SO₂R₈, un aryle éventuellement substitué par un (C₁-C₄)alkyle, un hydroxy, un alcoxy en C₁-C₂, un amino, un (di)alkyl(C₁-C₂)amino ; un aryle éventuellement substitué par un (C₁-C₄)alkyle , un hydroxy, un alcoxy en C₁-C₂, un amino, un (di)alkyl(C₁-C₂)amino ;
R₆ et R₇, identiques ou différents, peuvent représenter également un radical carboxamido CONR₈R₉; un sulfonyle SO₂R₈ ;
R₈ et R₉, identiques ou différents, représentent un atome d'hydrogène ; un radical alkyle en C₁-C₄ linéaire ou ramifié éventuellement substitué par un ou plusieurs hydroxy, alcoxy en C₁-C₂ :
R₃ et R₄ peuvent former avec l'atome d'azote auquel ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R₃ et R₄ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué.

2. Composition selon la revendication 1, dans laquelle R₁ et R₂ forment ensemble avec les atomes d'azotes auxquels ils sont rattachés un cycle à 5 ou 6 chaînons saturé ou insaturé éventuellement substitués.

3. Composition selon la revendication 1 ou 2, dans laquelle R₁ et R₂ forment ensemble avec les atomes d'azotes auxquels ils sont rattachés un cycle pyrazolidine, pyridazolidine, éventuellement substitué par un radical alkyle en C₁-C₄, un hydroxy, un (C₁-C₂)alcoxy, un carboxy, un carboxamido, un amino, un (di)alkyl(C₁-C₂)amino.

4. Composition selon l'une quelconque des revendications 1 à 3, dans laquelle R₁ et R₂ forment ensemble avec les atomes d'azotes auxquels ils sont rattachés un cycle pyrazolidine, pyridazolidine.

5. Composition selon l'une quelconque des revendications précédentes dans laquelle R₃ et R₄ sont choisis parmi un atome d'hydrogène ; un radical alkyle en C₁-C₄ linéaire ou ramifié éventuellement substitué par un ou plusieurs hydroxy, (C₁-C₂)alcoxy, amino, un (di)alkyl(C₁-C₂)amino ; un radical phényle éventuellement substitué par un radical hydroxy, amino, (C₁-C₂)alcoxy.

6. Composition selon l'une quelconque des revendications 1 à 5, dans laquelle R₃ et R₄ sont choisis parmi un atome d'hydrogène, un radical méthyle, éthyle, isopropyle, 2-hydroxyéthyle, 3-hydroxypropyle, 2-hydroxypropyle, 2-carboxyéthyle.

7. Composition selon la revendication 6 dans laquelle R3 et R4 représentent unatome d'hydrogène.

8. Composition selon l'une quelconque des revendications 1 à 4, dans laquelle R₃ et R₄ forment ensemble avec l'atome d'azote auquel ils sont rattachés un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl(C₁-C₂)amino, carboxy, carboxamido, alkyle en C₁-C₄ éventuellement substitué par un ou plusieurs hydroxy, amino, (di)alkylamino en C₁-C₂.

9. Composition selon l'une quelconque des revendications 1 à 4 et 8, dans laquelle R₃ et R₄ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthylpyrrolidine, la 3-hydroxypyrrolidine, la 3,4-dihydroxypyrrolidine, la 3-aminopyrrolidine, la 3-méthylaminopyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

10. Composition selon l'une quelconque des revendications 1 à 4, 8 et 9, dans laquelle R₃ et R₄ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthylhomopipérazine, la N β-hydroxyéthylhomopipérazine.

11. Composition selon l'une quelconque des revendications 1 à 4 et 8 à 10, dans laquelle R₃ et R₄ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

12. Composition selon l'une quelconque des revendications précédentes dans laquelle le composé de formule (I) ou un de ses sels d'addition est choisi parmi
2,3-diamino-6,7-dihydro-1H,5H-pyrazolo[1,2-a]pyrazol-1-one
2-amino-3-méthylamino-6,7-dihydro-1H,5H-pyrazolo[1,2-a]pyrazol-1-one
2-amino-3-diméthylamino-6,7-dihydro-1H,5H-pyrazolo[1,2-a]pyrazol-1-one
2-amino-3-éthylamino-6,7-dihydro-1H,5H-pyrazolo[1,2-a]pyrazol-1-one
2-amino-3-isopropylamino-6,7-dihydro-1H,5H-pyrazolo[1,2-a]pyrazol-1-one
2-amino-3-(2-hydroxyéthyl)amino-6,7-dihydro-1H,5H-pyrazolo[1,2-a]pyrazol-1-one
2-amino-3-(2-hydroxypropyl)amino-6,7-dihydro-1H,5H-pyrazolo[1,2-a]pyrazol-1-one
2-amino-3-bis(2-hydroxyéthyl)amino-6,7-dihydro-1H,5H-pyrazolo[1,2-a]pyrazol-1-one
2-amino-3-(pyrrolidin-1-yl) -6,7-dihydro-1H,5H-pyrazolo[1,2-a]pyrazol-1-one
2-amino-3-(3-hydroxy-pyrrolidin-1-yl) -6,7-dihydro-1H,5H-pyrazolo[1,2-a]pyrazol-1-one
2-amino-3-(pipéridin-1-yl)-6,7-dihydro-1 H,SH-pyrazolo[1,2-a]pyrazol-1-one
2,3-diamino-6-hydroxy-6,7-dihydro-1H,5H-pyrazolo[1,2-a]pyrazol-1-one
2,3-diamino-6-méthyl-6,7-dihydro-1H,5H-pyrazolo[1,2-a]pyrazol-1-one
2,3-diamino-6-diméthyl-6,7-dihydro-1H,5H-pyrazolo[1,2-a]pyrazol-1-one
2,3-diamino-5,6,7,8-tétrahydro-1 H,6H-pyridazino[1,2-a]pyrazol-1-one
2,3-diamino-5,8-dihydro-1H,6H-pyridazino[1,2-a]pyrazol-1-one.

13. Composition selon la revendication 1 à 12, dans laquelle le composé de formule (I) ou un de ses sels d'addition est choisi parmi
2,3-diamino-6,7-dihydro-1H,5H-pyrazolo[1,2-a]pyrazol-1-one
2-amino-3-isopropylamino-6,7-dihydro-1H,5H-pyrazolo[1,2-a]pyrazol-1-one
2-amino-3-éthylamino-6,7-dihydro-1 H, 5H-pyrazolo[1,2-a]pyrazol-1-one
2-amino-3-(2-hydroxyéthyl)amino-6,7-dihydro-1H,5H-pyrazolo[1,2-a]pyrazol-1-one
2-amino-3-diméthylamino-6,7-dihydro-1H,5H-pyrazolo[1,2-a]pyrazol-1-one
2-amino-3-(pyrrolidin-1-yl)-6,7-dihydro-1H,5H-pyrazolo[1,2-a]pyrazol-1-one
2,3-diamino-5,6,7,8-tétrahydro-1H,6H-pyridazino[1,2-a]pyrazol-1-one.

14. Composition selon l'une quelconque des revendications précédentes comprenant de plus un coupleur choisi parmi les méta-phénylènediamines, les méta-aminophénols, les méta-diphénols, les coupleurs naphtaléniques, les coupleurs hétérocycliques et leurs sels d'addition, ainsi que leurs mélanges.

15. Composition selon la revendication 14 dans laquelle la quantité de chacun des coupleurs est comprise entre 0,001 et 10 % en poids du poids total de la composition tinctoriale.

16. Composition selon l'une quelconque des revendications précédentes comprenant une base d'oxydation additionnelle choisie parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis dans l'une quelconque des revendications 1 à 13 et leurs sels d'addition, ainsi que leurs mélanges.

17. Composition selon la revendication 16 dans laquelle la quantité de chacune des bases d'oxydation est comprise entre 0,001 et 10 % en poids du poids total de la composition tinctoriale.

18. Procédé de teinture des fibres kératiniques, **caractérisé en ce qu'**une composition telle que définie dans l'une quelconque des revendications 1 à 17 est appliquée sur les fibres kératiniques en présence d'un agent oxydant pendant un temps suffisant pour développer la coloration désirée.

19. Procédé selon la revendication 18 dans lequel l'agent oxydant est choisi parmi le peroxyde d'hydrogène, le peroxyde d'urée, les bromates de métaux alcalins, les persels, les peracides et les enzymes oxydases.

20. Dispositif à plusieurs compartiments dans lequel un premier compartiment contient une composition tinctoriale telle que définie dans l'une quelconque des revendications 1 à 17 et un deuxième compartiment contient un agent oxydant.

21. Utilisation pour la teinture d'oxydation des fibres kératiniques d'une composition telle que définie dans l'une quelconque des revendications 1 à 17.

22. Dérivés d'amino-N,N-dihydro-pyrazolone de formule (I') ou leurs sels d'addition : 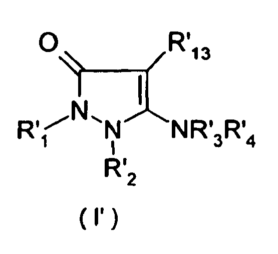 dans laquelle :
R'₁ et R'₂ forment avec les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R'₃ et R'₄ identiques ou différents, représentent
- un radical alkyle en C₁-C₆ linéaire ou ramifié éventuellement substitué par un ou plusieurs radicaux choisi dans le groupe constitué par un radical OR'₅, un radical NR'₆R'₇, un radical carboxy, un radical sulfonique, un radical carboxamido CONR'₆R'₇, un radical sulfonamido SO₂NR'₆R'₇, un hétéroaryle, un aryle éventuellement substitué par un groupe (C₁-C₄)alkyle, un hydroxy, un alcoxy en C₁-C₂, un amino, un (di)alkyl(C₁-C₂)amino;
- un radical aryle éventuellement substitué par un ou plusieurs (C₁-C₄)alkyle, hydroxy, alcoxy en C₁-C₂, amino, (di)alkyl(C₁-C₂)amino ;
- un radical hétéroaryle à 5 ou 6 chaînons, éventuellement substitué par un ou plusieurs radicaux choisis parmi (C₁-C₄)alkyle, (C₁-C₂)alcoxy;
R'₃ et R'₄ peuvent représenter également un atome d'hydrogène ;
R'₃ et R'₄ peuvent former avec l'atome d'azote auquel ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R'₃ et R'₄ peuvent également former ensemble avec l'atome d'azote auquel ils sont rattachés un hétérocycle à 5 ou 7 chaînons dont les atomes de carbone peuvent être remplacés par un atome d'oxygène ou d'azote éventuellement substitué ;
R'₅, R'₆ et R'₇, identiques ou différents, représentent un atome d'hydrogène ; un radical alkyle linéaire ou ramifié en C₁-C₄ éventuellement substitué par un ou plusieurs radicaux choisis dans le groupe constitué par un hydroxy, un alcoxy en C₁-C₂. un carboxamido CONR'₈R'₉, un sulfonyle SO₂R'₈, un aryle éventuellement substitué par un (C₁-C₄)alkyle, un hydroxy, un alcoxy en C₁-C₂, un amino, un (di)alkyl(C₁-C₂)amino ; un aryle éventuellement substitué par un (C₁-C₄)alkyle , un hydroxy, un alcoxy en C₁-C₂, un amino, un (di)alkyl(C₁-C₂)amino ;
R'₆ et R'₇, identiques ou différents, peuvent représenter également un radical carboxamido CONR'₈R'₉ ; un sulfonyle SO₂R'_{8;}
R'₈ et R'₉, identiques ou différents, représentent un atome d'hydrogène ; radical alkyle en C₁-C₄ linéaire ou ramifié éventuellement substitués par un ou plusieurs hydroxy, alcoxy en C₁-C₂ ;
R'₁₃ représente un groupe nitro, nitroso ou arylazo Ar-N=N-, le radical aryle Ar étant éventuellement substitué par un radical alkyle en C₁-C₄, amino, (di)alkyl(C₁-C₄)amino, alcoxy en C₁-C₂, sulfonique, carboxy, halogène.
sous réserve que
• R'₁₃ ne représente pas un groupe Ar-N=N- lorsque R'₃ et R'₄ représentent simultanément un atome d'hydrogène.

23. Dérivés de la diamino-N,N-dihydro-pyrazolone de formule (I") ou leurs sels d'addition : 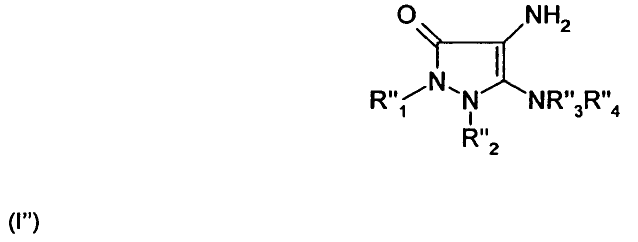 dans laquelle :
R"₁ et R"₂ forment avec les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R"₃ et R"₄, identiques ou différents, représentent :
- un radical alkyle en C₁-C₆ linéaire ou ramifié éventuellement substitué par un ou plusieurs radicaux choisi dans le groupe constitué par un radical OR"₅, un radical NR"₆R"₇, un radical carboxy, un radical sulfonique, un radical carboxamido CONR"₆R"₇, un radical sulfonamido SO₂NR"₆R"₇, un hétéroaryle, un aryle éventuellement substitué par un groupe (C₁-C₄)alkyle, un hydroxy, un alcoxy en C₁-C₂, un amino, un (di)alkyl(C₁-C₂)amino ;
- un radical aryle éventuellement substitué par un ou plusieurs (C₁-C₄)alkyle, hydroxy, alcoxy en C₁-C₂, amino, (di)alkyl(C₁-C₂)amino ;
- un radical hétéroaryle à 5 ou 6 chaînons, éventuellement substitué par un ou plusieurs radicaux choisis parmi (C₁-C₄)alkyle, (C₁-C₂)alcoxy ;
R"₃ et R"₄ peuvent représenter également un atome d'hydrogène ;
R"₅, R"₆ et R"₇, identiques ou différents, représentent un atome d'hydrogène ; un radical alkyle linéaire ou ramifié en C₁-C₄ éventuellement substitué par un ou plusieurs radicaux choisis dans le groupe constitué par un hydroxy, un alcoxy en C₁-C₂, un carboxamido CONR"₈R"₉, un sulfonyle SO₂R"₈, un aryle éventuellement substitué par un (C₁-C₄)alkyle, un hydroxy, un alcoxy en C₁-C₂, un amino, un (di)alkyl(C₁-C₂)amino ; un aryle éventuellement substitué par un (C₁-C₄)alkyle , un hydroxy, un alcoxy en C₁-C₂, un amino, un (di)alkyl(C₁-C₂)amino ;
R"₆ et R"₇, identiques ou différents, peuvent représenter également un radical carboxamido CONR"₈R"₉; un sulfonyle SO₂R"₈;
R"₈ et R"₉, identiques ou différents, représentent un atome d'hydrogène, radical alkyle en C₁-C₄ linéaire ou ramifié éventuellement substitués par un ou plusieurs hydroxy, alcoxy en C₁-C₂ ;
R"₃ et R"₄ peuvent former avec l'atome d'azote auquel ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
R"₃ et R"₄ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué.

24. Dérivés selon l'une quelconque des revendications 22 ou 23, pour lesquels, indépendamment les uns des autres, R"₁, R"₂ d'une part, R'₁ et R'₂ d'autre part, forment ensemble avec les atomes d'azotes auxquels ils sont rattachés, un cycle à 5 ou 6 chaînons, saturé ou insaturé, éventuellement substitué.

25. Dérivés selon l'une quelconque des revendications 22 à 24, pour lesquels R"₁, **R"₂** d'une part, R'₁ et R'₂ d'autre part, forment ensemble avec les atomes d'azotes auxquels ils sont rattachés, un cycle pyrazolidine, pyridazolidine, éventuellement substitué par un radical alkyle en C₁-C₄, un hydroxy, un (C₁-C₂)alcoxy, un carboxy, un carboxamido, un amino, un (di)alkyl(C₁-C₂)amino.

26. Dérivés selon l'une quelconque des revendications 22 à 25, pour lesquels R"₁, R"₂, d'une part, R'₁ et R'₂, d'autre part, forment ensemble avec les atomes d'azotes auxquels ils sont rattachés, un cycle pyrazolidine, pyridazolidine.

27. Dérivés selon l'une quelconque des revendications 22 à 26, pour lesquels R"₃, R"₄, R'₃ et R'₄, indépendamment les uns des autres, sont choisis parmi un atome d'hydrogène ; un radical alkyle en C₁-C₄ linéaire ou ramifié éventuellement substitué par un ou plusieurs hydroxy, (C₁-C₂)alcoxy, amino, un (di)alkyl(C₁-C₂)amino ; un radical phényle éventuellement substitué par un radical hydroxy, amino, (C₁-C₂)alcoxy.

28. Dérivés selon l'une quelconque des revendications 22 à 27, pour lesquels R"₃, R"₄, R'₃ et R'₄, indépendamment les uns des autres, sont choisis parmi un atome d'hydrogène, un méthyle, éthyle, isopropyle, 2-hydroxyéthyle, 3-hydroxypropyle, 2-hydroxypropyle, 2-carboxyéthyle.

29. Dérivés selon l'une quelconque des revendications 22 à 26, pour lesquels R"₃, R"₄ d'une part, R'₃ et R'₄, d'autre part, forment ensemble avec l'atome d'azote auquel ils sont rattachés, un cycle à 5 ou 7 chaînons, choisi parmi les hétérocycles pyrrolidine ; pipéridine ; homopipéridine ; pipérazine ; homopipérazine ; lesdits cycles pouvant être substitués par un ou plusieurs radicaux hydroxy ; amino ; (di)alkyl(C₁-C₂)amino ; carboxy; carboxamido ; alkyle en C₁-C₄ éventuellement substitué par un ou plusieurs hydroxy, amino, (di)alkylamino en C₁-C₂.

30. Dérivés selon l'une quelconque des revendications 22 à 26 et 29, pour lesquels R"₃, R"₄ d'une part, R'₃ et R'₄, d'autre part, forment ensemble avec l'atome d'azote auquel ils sont rattachés, un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la -3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

31. Dérivés selon l'une quelconque des revendications 22 à 26, 29 et 30, pour lesquels R"₃, R"₄ d'une part, R'₃ et R'₄, d'autre part, forment ensemble avec l'atome d'azote auquel ils sont rattachés, un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine 2-carboxylique, la pipéridine, l'hydroxypipéridine, l'homopipéridine, le diazépane, la N-méthyl homopipérazine, la N β-hydroxyéthylhomopipérazine.

32. Dérivés selon l'une quelconque des revendications 22 à 26 et 29 à 31 pour lesquels R"₃, R"₄ d'une part, R'₃ et R'₄, d'autre part, forment ensemble avec l'atome d'azote auquel ils sont rattachés, un cycle à 5 chaînons tel que la pyrrolidine, la 3-hydroxypyrrolidine, la 3-aminopyrrolidine, la 3-diméthylamino-pyrrolidine.

33. Procédé de préparation d'un composé de formule (I) tel que défini dans les compositions selon l'une quelconque des revendications 1 à 13, **caractérisé en ce que** l'on met en oeuvre les étapes suivantes :
a) étape 1 : on fait réagir un composé **a**
**R₁HN-NHR₂** **a**
avec un composé **b** : 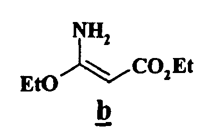 pour obtenir un composé 5-amino-1,2-dihydro-pyrazol-3-one **c** : 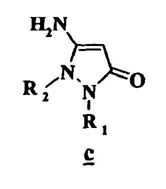
b) étape 2 : on fait réagir le dérivé **c** ainsi obtenu avec un sel d'aryldiazonium (Ar-N₂ ⁺Y⁻) pour obtenir un composé azoïque **f** : 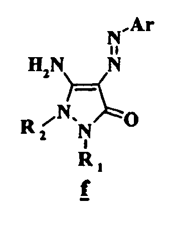
c) étape 3 : on effectue éventuellement une étape de fonctionalisation du groupement amine primaire du composé azoïque résultant **f** pour obtenir un composé **g** suivant : 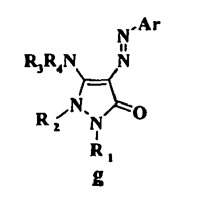
d) étape 4 : on effectue une réaction de réduction du composé azoïque **f** ou **g** pour obtenir, respectivement, un composé **e** ou **h** aminé :

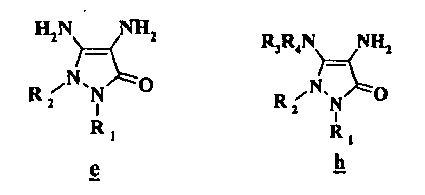

34. Procédé de préparation d'un composé de formule (I) tel que défini dans les compositions selon l'une quelconque des revendications 1 à 13, **caractérisé en ce que** l'on met en oeuvre les étapes suivantes :
a) étape 1 : on fait réagir un composé **a1** suivant : 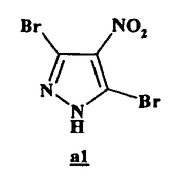 avec un composé **a2** : 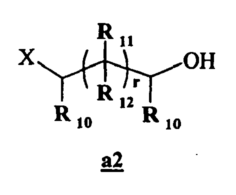 pour obtenir un composé **a3 :** 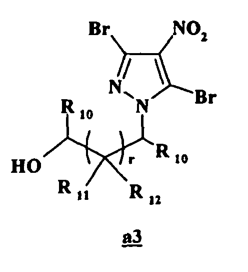 dans lesquelles :
le radical R₁₀ représente un atome d'hydrogène, un carboxy; un carboxamido ; un radical alkyle en C₁-C₄ éventuellement substitués par un ou plusieurs radicaux hydroxy, amino, (di)-alkylamino, alcoxy, carboxy, sulfonyle ;
les radicaux R₁₁ et R₁₂ représentent indépendamment les uns des autres des atomes d'hydrogène, d'halogène ; des radicaux amino ; (di)alkyl(C₁-C₄)amino ; hydroxy ; carboxy ; carboxamido; (C₁-C₂)alcoxy ; radical alkyle en C₁-C₄ éventuellement substitués par un ou plusieurs radicaux hydroxy, amino, (di)-alkylamino, alcoxy, carboxy, sulfonyle ;
X représente un atome d'halogène ou un alkylesulfonate.
r est un entier compris entre 1 et 3 ;
b) étape 2 : on fait réagir le composé **a3** avec une amine de formule NHR₃R₄ pour obtenir un composé **a4 :** 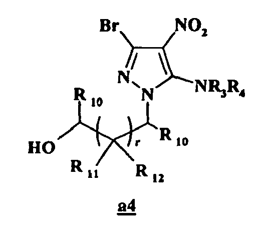
c) étape 3 : on fait réagir le composé **a4** avec au moins un halogénure d'alkylsulfonyle, d'arylsulfonyle ou de perfluoroalkylsulfonyle R-O₂S-X₁ (R représente un alkyle, un aryle ou un perfluoroalkyle, X₁ représente un halogène) dans un solvant aprotique pour obtenir un composé **a5 :** 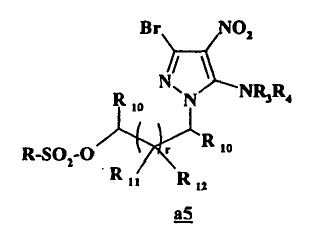
d) étape 4 : le composé **a5** résultant est ensuite chauffé dans un solvant de point d'ébullition compris entre 60°C et 190°C pour obtenir un composé **a6 :** 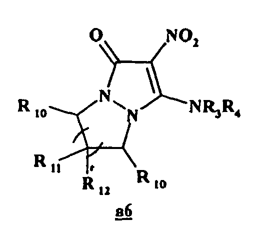
e) étape 5 : Le composé **a6** obtenu est réduit pour obtenir le composé **a7** de formule (III) ci-dessous :

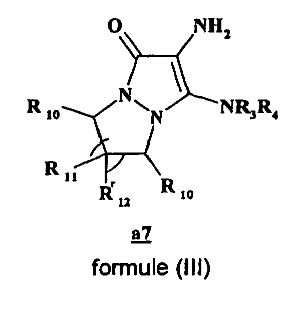

## Claims

1. Composition for dyeing keratinous fibres comprising, in an appropriate dyeing medium, as oxidation base, at least one diamino-N,N-dihydropyrazolone derivative of formula (I) or one of its addition salts or solvates: 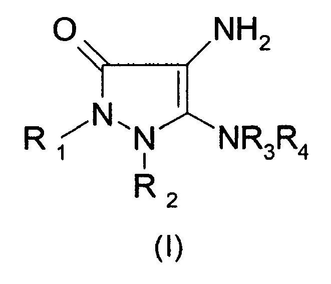 in which:
R₁ and R₂ form with the nitrogen atoms to which they are attached a saturated or unsaturated 5- to 7-membered heterocycle optionally substituted with one or more radicals chosen from the group consisting of halogen atoms, amino, (di)alkyl(C₁-C₄)amino, hydroxyl, carboxyl, carboxamido or (C₁-C₂)alkoxy radicals, C₁-C₄ alkyl radicals optionally substituted with one or more hydroxyl, amino, (di)alkylamino, alkoxy, carboxyl or sulphonyl radicals;
R₃ and R₄, which are identical or different, represent:
- a linear or branched C₁-C₆ alkyl radical optionally substituted with one or more radicals chosen from the group consisting of a radical OR₅, a radical NR₆R₇, a carboxyl radical, a sulphonic radical, a carboxamido radical CONR₆R₇, a sulphonamido radical SO₂NR₆R₇, a heteroaryl, an aryl optionally substituted with a (C₁-C₄)alkyl group, a hydroxyl, a C₁-C₂ alkoxy, an amino, a (di) alkyl (C₁-C₂) amino;
- an aryl radical optionally substituted with one or more (C₁-C₄) alkyl, hydroxyl, C₁-C₂ alkoxy, amino, (di) alkyl (C₁-C₂) amino;
- a 5- or 6-membered heteroaryl radical optionally substituted with one or more radicals chosen from (C₁-C₄) alkyl, (C₁-C₂) alkoxy;
R₃ and R₄ may also represent a hydrogen atom;
R₅, R₆ and R₇, which are identical or different, represent a hydrogen atom; a linear or branched C₁-C₄ alkyl radical optionally substituted with one or more radicals chosen from the group consisting of a hydroxyl, a C₁-C₂ alkoxy, a carboxamido CONR₈R₉, a sulphonyl SO₂R₈, an aryl optionally substituted with a (C₁-C₄)alkyl, a hydroxyl, a C₁-C₂ alkoxy, an amino, a (di)alkyl(C₁-C₂)amino; an aryl optionally substituted with a (C₁-C₄) alkyl, a hydroxyl, a C₁-C₂ alkoxy, an amino, a (di) alkyl (C₁-C₂) amino;
R₆ and R₇, which are identical or different, may also represent a carboxamido radical CONR₈R₉; a sulphonyl SO₂R₈;
R₈ and R₉, which are identical or different, represent a hydrogen atom; a linear or branched C₁-C₄ alkyl radical optionally substituted with one or more hydroxyl, C₁-C₂ alkoxy;
R₃ and R₄ may form with the nitrogen atom to which they are attached a saturated or unsaturated 5- to 7- membered heterocycle optionally substituted with one or more radicals chosen from the group consisting of halogen atoms, amino, (di)alkyl(C₁-C₄)amino, hydroxyl, carboxyl, carboxamido or (C₁-C₂) alkoxy radicals, C₁-C₄ alkyl radicals optionally substituted with one or more hydroxyl, amino, (di)alkylamino, alkoxy, carboxyl or sulphonyl radicals;
R₃ and R₄ may also form together with the nitrogen atom to which they are attached a 5- or 7-membered heterocycle whose carbon atoms may be replaced with an oxygen atom or optionally substituted nitrogen atom.

2. Composition according to Claim 1, in which R₁ and R₂ form together with the nitrogen atoms to which they are attached a saturated or unsaturated optionally substituted 5- or 6-membered ring.

3. Composition according to Claim 1 or 2, in which R₁ and R₂ form together with the nitrogen atoms to which they are attached a pyrazolidine or pyridazolidine ring optionally substituted with a C₁-C₄ alkyl radical, a hydroxyl, a (C₁-C₂) alkoxy, a carboxyl, a carboxamido, an amino, a (di) alkyl (C₁-C₂) amino.

4. Composition according to any one of Claims 1 to 3, in which R₁ and R₂ form together with the nitrogen atoms to which they are attached a pyrazolidine or pyridazolidine ring.

5. Composition according to any one of the preceding claims, in which R₃ and R₄ are chosen from a hydrogen atom; a linear or branched C₁-C₄ alkyl radical optionally substituted with one or more hydroxyl, (C₁-C₂)alkoxy, amino, a (di) alkyl (C₁-C₂) amino; a phenyl radical optionally substituted with a hydroxyl, amino or (C₁-C₂) alkoxy radical.

6. Composition according to any one of Claims 1 to 5, in which R₃ and R₄ are chosen from a hydrogen atom, a methyl, ethyl, isopropyl, 2-hydroxyethyl, 3-hydroxypropyl, 2-hydroxypropyl and 2-carboxyethyl radical.

7. Composition according to Claim 6, in which R₃ and R₄ represent a hydrogen atom.

8. Composition according to any one of Claims 1 to 4, in which R₃ and R₄ form together with the nitrogen atom to which they are attached a 5- or 7-membered ring chosen from the pyrrolidine, piperidine, homopiperidine, piperazine and homopiperazine heterocycles; it being possible for the said rings to be substituted with one or more hydroxyl, amino, (di) alkyl (C₁-C₂) amino, carboxyl or carboxamido radicals, or C₁-C₄ alkyl radicals optionally substituted with one or more hydroxyl, amino, C₁-C₂ (di)alkylamino.

9. Composition according to any one of Claims 1 to 4 and 8, in which R₃ and R₄ form together with the nitrogen atom to which they are attached a 5- or 7- membered ring chosen from pyrrolidine, 2,5-dimethylpyrrolidine, pyrrolidine-2-carboxylic acid, 3-hydroxypyrrolidine-2-carboxylic acid, 4-hydroxypyrrolidine-2-carboxylic acid, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido)-pyrrolidine, 2-hydroxymethylpyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 3-dimethylaminopyrrolidine, 4-amino-3-hydroxypyrrolidine, 3-hydroxy-4-(2-hydroxyethyl)aminopyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, homopiperazine, N-methylhomopiperazine, N-(2-hydroxyethyl)homopiperazine.

10. Composition according to any one of Claims 1 to 4, 8 and 9, in which R₃ and R₄ form together with the nitrogen atom to which they are attached a 5- or 7-membered ring chosen from pyrrolidine, 3-hydroxypyrrolidine, 3-aminopyrrolidine, 3-dimethylaminopyrrolidine, pyrrolidine-2-carboxylic acid, 3-hydroxypyrrolidine-2-carboxylic acid, piperidine, hydroxypiperidine, homopiperidine, diazepane, N-methylhomopiperazine, N-β-hydroxyethylhomopiperazine.

11. Composition according to any one of Claims 1 to 4 and 8 to 10, in which R₃ and R₄ form together with the nitrogen atom to which they are attached a 5-membered ring such as pyrrolidine, 3-hydroxypyrrolidine, 3-aminopyrrolidine, 3-dimethylaminopyrrolidine.

12. Composition according to any one of the preceding claims, in which the compound of formula (I) or one or its addition salts is chosen from
2,3-diamino-6,7-dihydro-1H,5H-pyrazolo[1,2-a]pyrazol-1-one
2-amino-3-methylamino-6,7-dihydro-1H,5H-pyrazolo[1,2-a]pyrazol-1-one
2-amino-3-dimethylamino-6,7-dihydro-1H,5H-pyrazolo-[1,2-a]pyrazol-1-one
2-amino-3-ethylamino-6,7-dihydro-1H,5H-pyrazolo-[1,2-a]pyrazol-1-one
2-amino-3-isopropylamino-6,7-dihydro-1H,5H-pyrazolo-[1,2-a]pyrazol-1-one
2-amino-3-(2-hydroxyethyl)amino-6,7-dihydro-1H,5H-pyrazolo[1,2-a]pyrazol-1-one
2-amino-3-(2-hydroxypropyl)amino-6,7-dihydro-1H,5H-pyrazolo[1,2-a]pyrazol-1-one
2-amino-3-bis(2-hydroxyethyl)amino-6,7-dihydro-1H,5H-pyrazolo[1,2-a]pyrazol-1-one
2-amino-3-(pyrrolidin-1-yl)-6,7-dihydro-1H,5H-pyrazolo-[1,2-a]pyrazol-1-one
2-amino-3-(3-hydroxypyrrolidin-1-yl)-6,7-dihydro-1H,5H-pyrazolo[1,2-a]pyrazol-1-one
2-amino-3-(piperidin-1-yl)-6,7-dihydro-1H,5H-pyrazolo-[1,2-a]pyrazol-1-one.
2,3-diamino-6-hydroxy-6,7-dihydro-1H,5H-pyrazolo-[1,2-a]pyrazol-1-one
2,3-diamino-6-methyl-6,7-dihydro-1H,5H-pyrazolo[1,2-a]-pyrazol-1-one
2,3-diamino-6-dimethyl-6,7-dihydro-1H,5H-pyrazolo-[1,2-a]pyrazol-1-one
2,3-diamino-5,6,7,8-tetrahydro-1H,6H-pyridazino-[1,2-a]pyrazol-1-one
2,3-diamino-5,8-dihydro-1H,6H-pyridazino[1,2-a]pyrazol-1-one.

13. Composition according to Claims 1 to 12, in which the compound of formula (I) or one of its addition salts is chosen from
2,3-diamino-6,7-dihydro-1H,5H-pyrazolo[1,2-a]pyrazol-1-one
2-amino-3-isopropylamino-6,7-dihydro-1H,5H-pyrazolo-[1,2-a]pyrazol-1-one
2-amino-3-ethylamino-6,7-dihydro-1H,5H-pyrazolo[1,2-a]-pyrazol-1-one
2-amino-3-(2-hydroxyethyl)amino-6,7-dihydro-1H,5H-pyrazolo[1,2-a]pyrazol-1-one
2-amino-3-dimethylamino-6,7-dihydro-1H,5H-pyrazolo[1,2-a]pyrazol-1-one
2-amino-3-(pyrrolidin-1-yl)-6,7-dihydro-1H,5H-pyrazolo-[1,2-a]pyrazol-1-one
2,3-diamino-5,6,7,8-tetrahydro-1H,6H-pyridazino-[1,2-a]pyrazol-1-one.

14. Composition according to any one of the preceding claims, further comprising a coupler chosen from meta-phenylenediamines, meta-aminophenols, meta-diphenols, naphthalene couplers, heterocyclic couplers and their addition salts, and mixtures thereof.

15. Composition according to Claim 14, in which the quantity of each of the couplers is between 0.001 and 10% by weight of the total weight of the dyeing composition.

16. Composition according to any one of the preceding claims, comprising an additional oxidation base chosen from para-phenylenediamines, bisphenylalkylenediamines, para-aminophenols, bis-para-aminophenols, ortho-aminophenols, ortho-phenylenediamines, heterocyclic bases different from the derivatives of formula (I) as defined in any one of Claims 1 to 13 and their addition salts, and mixtures thereof.

17. Composition according to Claim 16, in which the quantity of each of the oxidation bases is between 0.001 and 10% by weight of the total weight of the dyeing composition.

18. Method for dyeing keratinous fibres, **characterized in that** a composition as defined in any one of Claims 1 to 17 is applied to the keratinous fibres in the presence of an oxidizing agent, for a period which is sufficient to develop the desired colour.

19. Method according to Claim 18, in which the oxidizing agent is chosen from hydrogen peroxide, urea peroxide, alkali metal bromates, persalts, peracids and oxidase enzymes.

20. Multicompartment device in which a first compartment contains a dyeing composition as defined in any one of Claims 1 to 17 and a second compartment contains an oxidizing agent.

21. Use, for the oxidation dyeing of keratinous fibres, of a composition as defined in any one of Claims 1 to 17.

22. Amino-N,N-dihydropyrazolone derivatives of formula (I') or their addition salts: 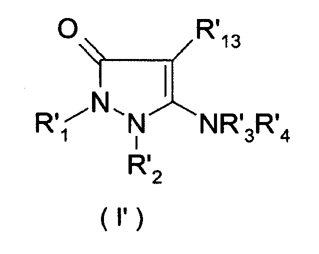 in which:
R'₁, and R'₂ form with the nitrogen atoms to which they are attached a saturated or unsaturated 5- to 7-membered heterocycle optionally substituted with one or more radicals chosen from the group consisting of halogen atoms, amino, (di)alkyl(C₁-C₄)amino, hydroxyl, carboxyl, carboxamido or (C₁-C₂) alkoxy radicals, C₁-C₄ alkyl radicals optionally substituted with one or more hydroxyl, amino, (di)alkylamino, alkoxy, carboxyl or sulphonyl radicals;
R'₃ and R'₄, which are identical or different, represent:
- a linear or branched C₁-C₆ alkyl radical optionally substituted with one or more radicals chosen from the group consisting of a radical OR'₅, a radical NR'₆R'₇, a carboxyl radical, a sulphonic radical, a carboxamido radical CONR'₆R'₇, a sulphonamido radical SO₂NR'₆R'₇, a heteroaryl, an aryl optionally substituted with a (C₁-C₄) alkyl group, a hydroxyl, a C₁-C₂ alkoxy, an amino, a (di) alkyl (C₁-C₂) amino;
- an aryl radical optionally substituted with one or more (C₁-C₄)alkyl, hydroxyl, C₁-C₂ alkoxy, amino, (di) alkyl (C₁-C₂) amino;
- a 5- or 6-membered heteroaryl radical optionally substituted with one or more radicals chosen from (C₁-C₄) alkyl, (C₁-C₂) alkoxy;
R'₃ and R'₄ may also represent a hydrogen atom;
R'₃ and R'₄ may form with the nitrogen atom to which they are attached a saturated or unsaturated 5- to 7- membered heterocycle optionally substituted with one or more radicals chosen from the group consisting of halogen atoms, amino, (di)alkyl(C₁-C₄)amino, hydroxyl, carboxyl, carboxamido or (C₁-C₂)alkoxy radicals, C₁-C₄ alkyl radicals optionally substituted with one or more hydroxyl, amino, (di)alkylamino, alkoxy, carboxyl or sulphonyl radicals;
R'₃ and R'₄ may also form together with the nitrogen atom to which they are attached a 5- or 7-membered heterocycle whose carbon atoms may be replaced with an oxygen atom or optionally substituted nitrogen atom;
R'₅, R'₆ and R'₇, which are identical or different, represent a hydrogen atom; a linear or branched C₁-C₄ alkyl radical optionally substituted with one or more radicals chosen from the group consisting of a hydroxyl, a C₁-C₂ alkoxy, a carboxamido CONR'₈R'₉, a sulphonyl SO₂R'₈, an aryl optionally substituted with a (C₁-C₄) alkyl, a hydroxyl, a C₁-C₂ alkoxy, an amino, a (di)alkyl(C₁-C₂)amino; an aryl optionally substituted with a (C₁-C₄) alkyl, a hydroxyl, a C₁-C₂ alkoxy, an amino, a (di)alkyl(C₁₋C₂)amino;
R'₆ and R'₇, which are identical or different, may also represent a carboxamido radical CONR'₈R'₉; a sulphonyl SO₂R'₈;
R'₈ and R'₉, which are identical or different, represent a hydrogen atom; a linear or branched C₁-C₄ alkyl radical optionally substituted with one or more hydroxyl, C₁-C₂ alkoxy;
R'₁₃ represents a nitro, nitroso or arylazo Ar-N=N-group, the aryl radical Ar being optionally substituted with a C₁-C₄ alkyl, amino, (di) alkyl (C₁-C₄) amino, C₁-C₂ alkoxy, sulphonic, carboxyl or halogen radical; provided that
• R'₁₃ does not represent a group Ar-N=N- when R'₃ and R'₄ simultaneously represent a hydrogen atom.

23. Diamino-N,N-dihydropyrazolone derivatives of formula (I") or their addition salts: 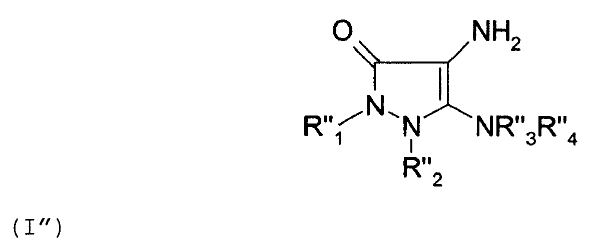 in which:
R"₁ and R"₂ form with the nitrogen atoms to which they are attached a saturated or unsaturated 5- to 7-membered heterocycle optionally substituted with one or more radicals chosen from the group consisting of halogen atoms, amino, (di)alkyl(C₁-C₄)amino, hydroxyl, carboxyl, carboxamido or (C₁-C₂) alkoxy radicals, C₁-C₄ alkyl radicals optionally substituted with one or more hydroxyl, amino, (di)alkylamino, alkoxy, carboxyl or sulphonyl radicals;
R"₃ and R"₄, which are identical or different, represent:
- a linear or branched C₁-C₆ alkyl radical optionally substituted with one or more radicals chosen from the group consisting of a radical OR"₅, a radical NR"₆R"₇, a carboxyl radical, a sulphonic radical, a carboxamido radical CONR"₆R"₇, a sulphonamido radical SO₂NR"₆R"₇, a heteroaryl, an aryl optionally substituted with a (C₁-C₄) alkyl group, a hydroxyl, a C₁-C₂ alkoxy, an amino, a (di) alkyl (C₁-C₂) amino;
- an aryl radical optionally substituted with one or more (C₁-C₄)alkyl, hydroxyl, C₁-C₂ alkoxy, amino, (di) alkyl (C₁-C₂) amino;
- a 5- or 6-membered heteroaryl radical optionally substituted with one or more radicals chosen from (C₁-C₄) alkyl, (C₁-C₂) alkoxy;
R"₃ and R"₄ may also represent a hydrogen atom;
R"₅, R"6 and R"₇, which are identical or different, represent a hydrogen atom; a linear or branched C₁-C₄ alkyl radical optionally substituted with one or more radicals chosen from the group consisting of a hydroxyl, a C₁-C₂ alkoxy, a carboxamido CONR"₈R"₉, a sulphonyl SO₂R"₈, an aryl optionally substituted with a (C₁-C₄) alkyl, a hydroxyl, a C₁-C₂ alkoxy, an amino, a (di)alkyl(C₁-C₂)amino; an aryl optionally substituted with a (C₁-C₄) alkyl, a hydroxyl, a C₁-C₂ alkoxy, an amino, a (di)alkyl(C₁-C₂)amino;
R"₆ and R"₇, which are identical or different, may also represent a carboxamido radical CONR"₈R"₉; a sulphonyl SO₂R"₈;
R"₈ and R"₉, which are identical or different, represent a hydrogen atom; a linear or branched C₁-C₄ alkyl radical optionally substituted with one or more hydroxyl, C₁-C₂ alkoxy;
R"₃ and R"₄ may form with the nitrogen atom to which they are attached a saturated or unsaturated 5- to 7- membered heterocycle optionally substituted with one or more radicals chosen from the group consisting of halogen atoms, amino, (di)alkyl(C₁-C₄)amino, hydroxyl, carboxyl, carboxamido or (C₁-C₂)alkoxy radicals, C₁-C₄ alkyl radicals optionally substituted with one or more hydroxyl, amino, (di)alkylamino, alkoxy, carboxyl or sulphonyl radicals;
R"₃ and R"₄ may also form together with the nitrogen atom to which they are attached a 5- or 7-membered heterocycle whose carbon atoms may be replaced with an oxygen atom or optionally substituted nitrogen atom.

24. Derivatives according to either of Claims 22 and 23, for which, independently of each other, R"₁, R"₂ on the one hand, R'₁ and R'₂ on the other hand, form together with the nitrogen atoms to which they are attached a saturated or unsaturated, optionally substituted 5- or 6-membered ring.

25. Derivatives according to any one of Claims 22 to 24, for which R"₁, R"₂ on the one hand, R'₁ and R'₂ on the other hand, form together with the nitrogen atoms to which they are attached a pyrazolidine or pyridazolidine ring optionally substituted with a C₁-C₄ alkyl radical, a hydroxyl, a (C₁-C₂)alkoxy, a carboxyl, a carboxamido, an amino, a (di)alkyl(C₁-C₂)amino.

26. Derivatives according to any one of Claims 22 to 25, for which R"₁, R"₂ on the one hand, R'₁ and R'₂ on the other hand, form together with the nitrogen atoms to which they are attached a pyrazolidine or pyridazolidine ring.

27. Derivatives according to any one of Claims 22 to 26, for which R"₃, R"₄, R'₃ and R'₄, independently of each other, are chosen from a hydrogen atom; a linear or branched C₁-C₄ alkyl radical optionally substituted with one or more hydroxyl, (C₁-C₂) alkoxy, amino, a (di) alkyl (C₁-C₂) amino; a phenyl radical optionally substituted with a hydroxyl, amino or (C₁-C₂)alkoxy radical.

28. Derivatives according to any one of Claims 22 to 27, for which R"₃, R"₄, R'₃ and R'₄, independently of each other, are chosen from a hydrogen atom, a methyl, ethyl, isopropyl, 2-hydroxyethyl, 3-hydroxypropyl, 2-hydroxypropyl and 2-carboxyethyl.

29. Derivatives according to any one of Claims 22 to 26, for which R"₃, R"₄ on the one hand, R'₃ and R'₄ on the other hand, form together with the nitrogen atom to which they are attached a 5- or 7-membered ring chosen from the pyrrolidine, piperidine, homopiperidine, piperazine and homopiperazine heterocycles; it being possible for the said rings to be substituted with one or more hydroxyl, amino,(di)alkyl(C₁-C₂)amino, carboxyl or carboxamido radicals, or C₁-C₄ alkyl radicals optionally substituted with one or more hydroxyl, amino, C₁-C₂ (di)alkylamino.

30. Derivatives according to any one of Claims 22 to 26 and 29, for which R"₃, R"₄ on the one hand, R'₃ and R'₄ on the other hand, form together with the nitrogen atom to which they are attached a 5- or 7-membered ring chosen from pyrrolidine, 2,5-dimethylpyrrolidine, pyrrolidine-2-carboxylic acid, 3-hydroxypyrrolidine-2-carboxylic acid, 4-hydroxypyrrolidine-2-carboxylic acid, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido)pyrrolidine, 2-hydroxymethylpyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 3-dimethylaminopyrrolidine, 4-amino-3-hydroxypyrrolidine, 3-hydroxy-4-(2-hydroxyethyl)aminopyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, homopiperazine, N-methylhomopiperazine, N-(2-hydroxyethyl)homopiperazine.

31. Derivatives according to any one of Claims 22 to 26, 29 and 30, for which R"₃, R"₄ on the one hand, R'₃ and R'₄ on the other hand, form together with the nitrogen atom to which they are attached a 5- or 7-membered ring chosen from pyrrolidine, 3-hydroxypyrrolidine, 3-aminopyrrolidine, 3-dimethylaminopyrrolidine, pyrrolidine-2-carboxylic acid, 3-hydroxypyrrolidine-2-carboxylic acid, piperidine, hydroxypiperidine, homopiperidine, diazepane, N-methylhomopiperazine, N-β-hydroxyethylhomopiperazine.

32. Derivatives according to any one of Claims 22 to 26 and 29 to 31, for which R"₃, R"₄ on the one hand, R'₃ and R'₄ on the other hand, form together with the nitrogen atom to which they are attached a 5-membered ring such as pyrrolidine, 3-hydroxypyrrolidine, 3-aminopyrrolidine, 3-dimethylaminopyrrolidine.

33. Method for preparing a compound of formula (I) as defined in the compositions according to any one of Claims 1 to 13, **characterized in that** the following steps are carried out:
a) step 1: a compound **a**
**R₁HN-NHR₂** **a**
is reacted with a compound **b**: 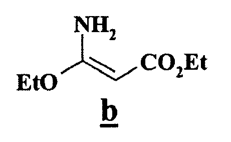 to give a compound 5-amino-1,2-dihydropyrazol-3-one **c**: 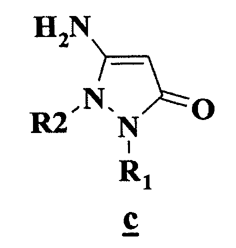
b) step 2: the derivative **c** thus obtained is reacted with an aryldiazonium salt (Ar-N₂⁺Y⁻) to give an azo compound **f**: 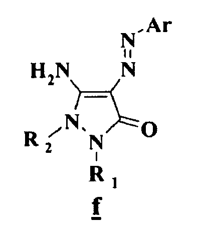
c) step 3: a step of functionalizing the primary amine group of the resulting azo compound **f** is optionally carried out in order to give the following compound **g**: 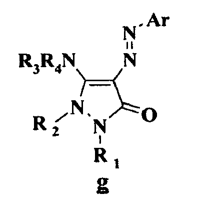
d) step 4: a reaction of reduction of the azo compound **f** or **g** is carried out in order to obtain an amine-containing compound **e** or **h** respectively:

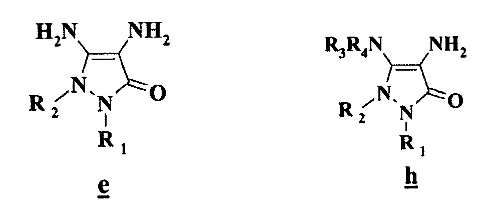

34. Method for preparing a compound of formula (I) as defined in the compositions according to any one of Claims 1 to 13, **characterized in that** the following steps are carried out:
a) step 1: the following compound **a1**: 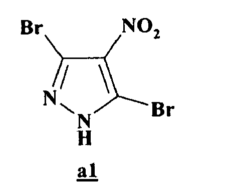 is reacted with a compound **a2:** 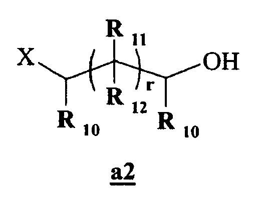 to give a compound **a3:** 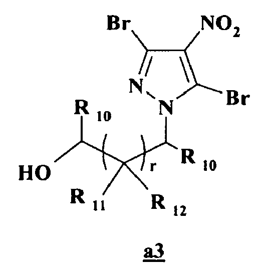 in which:
the radical R₁₀ represents a hydrogen atom, a carboxyl; a carboxamido; a C₁-C₄ alkyl radical optionally substituted with one or more hydroxyl, amino, (di)alkylamino, alkoxy, carboxyl and sulphonyl radicals;
the radicals R₁₁ and R₁₂ represent independently of each other hydrogen or halogen atoms; an amino radical; a (di)alkyl(C₁-C₄)amino radical; a hydroxyl radical; a carboxyl radical; a carboxamido radical; a (C₁-C₂) alkoxy radical; a C₁-C₄ alkyl radical optionally substituted with one or more hydroxyl, amino, (di)alkylamino, alkoxy, carboxyl or sulphonyl radicals;
X represents a halogen atom or an alkyl sulphonate;
r is an integer between 1 and 3;
b) step 2: the compound **a3** is reacted with an amine of formula NHR₃R₄ to give a compound **a4:** 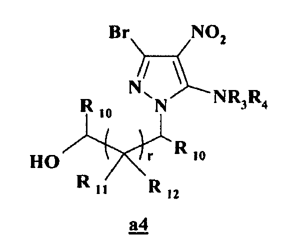
c) step 3: the compound **a4** is reacted with at least one alkylsulphonyl, arylsulphonyl or perfluoroalkylsulphonyl halide R-O₂S-X₁ (R represents an alkyl, an aryl or a perfluoroalkyl, X₁ represents a halogen), in an aprotic solvent in order to give a compound **a5**: 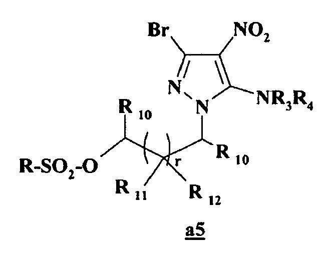
d) step 4: the resulting compound **a5** is then heated in a solvent having a boiling point of between 60°C and 190°C to give a compound **a6**: 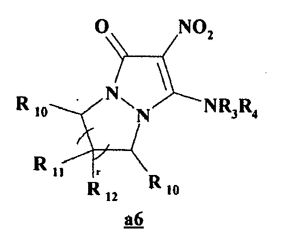
e) step 5: the compound **a6** obtained is reduced to give the compound **a7** of formula (III) below:

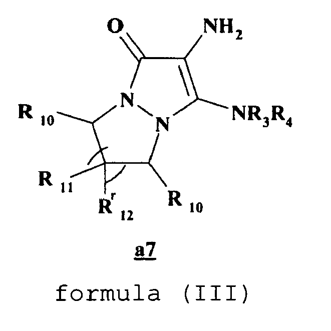

## Patentansprüche

1. Zusammensetzung zum Färben von Keratinfasern, die in einem zum Färben geeigneten Medium mindestens ein Diamino-N,N-dihydro-pyrazolonderivat der Formel (I) oder eines seiner Additionssalze oder Solvate als Oxidationsbase enthält: 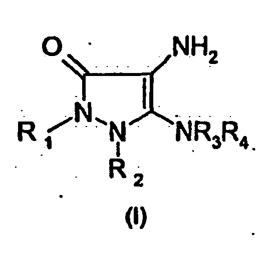 in der Formel:
R₁ und R₂ bilden mit den Stickstoffatomen, an die sie gebunden sind, einen gesättigten oder ungesättigten, 5- bis 7-gliedrigen Heterocyclus, der gegebenenfalls mit einer oder mehreren Gruppen substituiert ist, die ausgewählt sind unter den Halogenatomen, den Gruppen Amino, (Di)alkyl(C₁₋₄)amino, Hydroxy, Carboxy, Carboxamido, C₁₋₂-Alkoxy, den C₁₋₄-Alkylgruppen, die gegebenenfalls mit einer oder mehreren Gruppen Hydroxy, Amino, (Di)alkylamino, Alkoxy, Carboxy, Sulfonyl substituiert sind;
R₃ et R₄, die gleich oder verschieden sind, bedeuten:
- eine geradkettige oder verzweigte C₁₋₆-Alkylgruppe, die gegebenenfalls mit einer oder mehreren Gruppen substituiert ist, die ausgewählt sind unter einer Gruppe OR₅, einer Gruppe NR₆R₇, einer Carboxygruppe, einer Sulfonsäuregruppe, einer Carboxamidogruppe CONR₆R₇, einer Sulfonamidogruppe SO₂NR₆R₇, einer Heteroarylgruppe, einer Arylgruppe, die gegebenenfalls substituiert ist mit C₁₋₄-Alkyl, Hydroxy, C₁₋₂-Alkoxy, Amino, (Di)alkyl(C₁₋₂)amino;
- eine Arylgruppe , die gegebenenfalls mit einer oder mehreren Gruppen C₁₋₄-Alkyl, Hydroxy, C₁₋₂-Alkoxy, Amino, (Di)alkyl(C₁₋₂) amino substituiert ist;
- eine 5- oder 6-gliedrige Heteroarylgruppe, die gegebenenfalls mit einer oder mehreren Gruppen substituiert ist, die unter C₁₋₄-Alkyl, C₁₋₂-Alkoxy ausgewählt sind;
R₃ et R₄ können auch ein Wasserstoffatom bedeuten;
R₅, R₆ et R₇, die gleich oder verschieden sind, bedeuten ein Wasserstoffatom; eine geradkettige oder verzweigte C₁₋₄-Alkylgruppe, die gegebenenfalls mit einer oder mehreren Gruppen substituiert ist, die ausgewählt sind unter Hydroxy, C₁₋₂-Alkoxy, Carboxamido CONR₈R₉, Sulfonyl SO₂R₈, einer Arylgruppe, die gegebenenfalls substituiert ist mit C₁₋₄-Alkyl, Hydroxy, C₁₋₂-Alkoxy, Amino, (Di)alkyl(C₁₋₂)amino; eine Arylgruppe, die gegebenenfalls substituiert ist mit C₁₋₄-Alkyl, Hydroxy, C₁₋₂-Alkoxy, Amino, (Di)alkyl(C₁₋₂)amino;
R₆ et R₇, die gleich oder verschieden sind, können auch eine Carboxamidogruppe CONR₈R₉, eine Sulfonylgruppe SO₂R₈ bedeuten;
R₈ et R₉, die gleich oder verschieden sind, bedeuten ein Wasserstoffatom; eine geradkettige oder verzweigte C₁₋₄-Alkylgruppe, die gegebenenfalls mit einer oder mehreren Gruppen Hydroxy, C₁₋₂-Alkoxy substituiert ist;
R₃ et R₄ können mit dem Stickstoffatom, an das sie gebunden sind, einen gesättigten oder ungesättigten, 5- bis 7-gliedrigen Heterocyclus bilden, der gegebenenfalls mit einer oder mehreren Gruppen substituiert ist, die ausgewählt sind unter den Halogenatomen, den Gruppen Amino, (Di)alkyl(C₁₋₄)amino, Hydroxy, Carboxy, Carboxamido, C₁₋₂-Alkoxy, den C₁₋₄-Alkylgruppen, die gegebenenfalls mit einer oder mehreren Gruppen Hydroxy, Amino, (Di)alkylamino, Alkoxy, Carboxy, Sulfonyl substituiert sind;
R₃ et R₄ können auch gemeinsam mit dem Stickstoffatom, an das sie gebunden sind, einen 5- oder 7-gliedrigen Heterocyclus bilden, bei dem die Kohlenstoffatome durch ein Sauerstoffatom oder Stickstoffatom ersetzt sein können, das gegebenenfalls substituiert ist.

2. Zusammensetzung nach Anspruch 1, bei der die Gruppen R₁ et R₂ gemeinsam mit den Stickstoffatomen, an die sie gebunden sind, einen gesättigten oder ungesättigten, 5- oder 6-gliedrigen Ring bilden, der gegebenenfalls substituiert ist.

3. Zusammensetzung nach Anspruch 1 oder 2, bei der die Gruppen R₁ et R₂ gemeinsam mit den Stickstoffatomen, an die sie gebunden sind, einen Pyrazolidinring oder Pyridazolidinring bilden, die gegebenenfalls mit (C₁₋₄)Alkyl, Hydroxy, (C₁₋₂)Alkoxy, Carboxy, Carboxamido, Amino, (Di)alkyl(C₁₋₂)amino substituiert sind.

4. Zusammensetzung nach einem der Ansprüche 1 bis 3, bei der die Gruppen R₁ et R₂ gemeinsam mit den Stickstoffatomen, an die sie gebunden sind, einen Pyrazolidinring oder Pyridazolidinring bilden.

5. Zusammensetzung nach einem der vorhergehenden Ansprüche, bei der die Gruppen R₃ et R₄ unter einem Wasserstoffatom; einer geradkettigen oder verzweigten Alkyl(C₁₋₄)gruppe, die gegebenenfalls mit einer oder mehreren Gruppen Hydroxy, (C₁₋₂)Alkoxy, Amino, (Di)alkyl(C₁₋₂)amino substituiert ist; einer Phenylgruppe, die gegebenenfalls mit Hydroxy, Amino, (C₁₋₂)Alkoxy substituiert ist, ausgewählt sind.

6. Zusammensetzung nach einem der Ansprüche 1 bis 5, bei der die Gruppen R₃ et R₄ unter einem Wasserstoffatom, Methyl, Ethyl, Isopropyl, 2-Hydroxyethyl, 3-Hydroxypropyl, 2-Hydroxypropyl, 2-Carboxyethyl ausgewählt sind.

7. Zusammensetzung nach Anspruch 6, bei der die Gruppen R₃ et R₄ ein Wasserstoffatom bedeuten.

8. Zusammensetzung nach einem der Ansprüche 1 bis 4, bei der die Gruppen R₃ et R₄ gemeinsam mit dem Stickstoffatom, an das sie gebunden sind, einen 5- oder 7-gliedrigen Ring bilden, der unter den Heterocyclen Pyrrolidin, Piperidin, Homopiperidin, Piperazin, Homopiperazin ausgewählt ist; wobei diese Ringe gegebenenfalls substituiert sein können mit einer oder mehreren Gruppen Hydroxy, Amino, (Di)alkyl(C₁₋₂)amino, Carboxy, Carboxamido, (C₁₋₄)Alkyl, wobei diese Gruppe gegebenenfalls mit einer oder mehreren Gruppen Hydroxy, Amino, (Di)alkyl(C₁₋₂)amino substituiert sein kann.

9. Zusammensetzung nach einem der Ansprüche 1 bis 4 und 8, bei der die Gruppen R₃ et R₄ gemeinsam mit dem Stickstoffatom, an das sie gebunden sind, einen 5- oder 7-gliedrigen Ring bilden, der unter Pyrrolidin, 2,5-Dimethylpyrrolidin, Pyrrolidin-2-carbonsäure, 3-Hydroxypyrrolidin-2-carbonsäure, 4-Hydroxypyrrolidin-2-carbonsäure, 2,4-Dicarboxypyrrolidin, 3-Hydroxy-2-hydroxy-methylpyrrolidin, 2-Carboxamidopyrrolidin, 3-Hydroxy-2-carbox-amidopyrrolidin, 2-(Diethylcarboxamido)pyrrolidin, 2-Hydroxymethyl-pyrrolidin, 3,4-Dihydroxy-2-hydroxymethyl-pyrrolidin, 3-Hydroxypyrrolidin, 3,4-Dihydroxypyrrolidin, 3-Aminopyrrolidin, 3-Methylaminopyrrolidin, 3-Dimethylamino-pyrrolidin, 4-Amino-3-hydroxypyrrolidin, 3-Hydroxy-4-(2-hydroxyethyl)aminopyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxy-homo-piperidin, 2-Carboxamidohomopiperidin, Homopiperazin, N-Methyl-homopiperazin, N-(2-Hydroxyethyl)homopiperazin ausgewählt ist.

10. Zusammensetzung nach einem der Ansprüche 1 bis 4, 8 und 9, bei der die Gruppen R₃ et R₄ gemeinsam mit dem Stickstoffatom, an das sie gebunden sind, einen 5- oder 7-gliedrigen Ring bilden, der unter Pyrrolidin, 3-Hydroxypyrrolidin, 3-Aminopyrrolidin, 3-Dimethylamino-pyrrolidin, Pyrrolidin-2-carbonsäure, 3-Hydroxypyrrolidin-2-carbonsäure, Piperidin, Hydroxypiperidin, Homopiperidin, Diazepan, N-Methylhomopiperazin, N-β-Hydroxyethylhomopiperazin ausgewählt ist.

11. Zusammensetzung nach einem der Ansprüche 1 bis 4 und 8 bis 10, bei der die Gruppen R₃ et R₄ gemeinsam mit dem Stickstoffatom, an das sie gebunden sind, einen 5-gliedrigen Ring bilden, wie Pyrrolidin, 3-Hydroxypyrrolidin, 3-Aminopyrrolidin, 3-Dimethylaminopyrrolidin.

12. Zusammensetzung nach einem der vorhergehenden Ansprüche, bei der die Verbindung der Formel (I) oder eines ihrer Additionssalze ausgewählt ist unter:
2,3-Diamino-6,7-dihydro-1H,5H-pyrazolo[1,2-a]pyrazol-1-on
2-Amino-3-methylamino-6,7-dihydro-1H,5H-pyrazolo[1,2-a]-pyrazol-1-on
2-Amino-3-dimethylamino-6,7-dihydro-1H,5H-pyrazolo[1,2-a]-pyrazol-1-on
2-Amino-3-ethylamino-6,7-dihydro-1H,5H-pyrazolo[1,2-a]-pyrazol-1-on
2-Amino-3-isopropylamino-6,7-dihydro-1H,5H-pyrazolo[1,2-a]-pyrazol-1-on
2-Amino-3-(2-hydroxyethyl)amino-6,7-dihydro-1H,5H-pyrazolo[1,2-a]pyrazol-1-on
2-Amino-3-(2-hydroxypropyl)amino-6,7-dihydro-1H,5H-pyrazolo[1,2-a]pyrazol-1-on
2-Amino-3-bis(2-hydroxyethyl)amino-6,7-dihydro-1H,5H-pyrazolo[1,2-a]pyrazol-1-on
2-Amino-3-(pyrrolidin-1-yl) -6,7-dihydro-1H,5H-pyrazolo[1,2-a]-pyrazol-1-on
2-Amino-3-(3-hydroxy-pyrrolidin-1-yl) -6,7-dihydro-1H,5H-pyrazolo[1,2-a]pyrazol-1-on
2-Amino-3-(piperidin-1-yl)-6,7-dihydro-1H,5H-pyrazolo[1,2-a]-pyrazol-1-on
2,3-Diamino-6-hydroxy-6,7-dihydro-1H,5H-pyrazolo[1,2-a]-pyrazol-1-on
2,3-Diamino-6-methyl-6,7-dihydro-1H,5H-pyrazolo[1,2-a]-pyrazol-1-on
2,3-Diamino-6-dimethyl-6,7-dihydro-1H,5H-pyrazolo[1,2-a]-pyrazol-1-on
2,3-Diamino-5,6,7,8-tetrahydro-1H,6H-pyridazino[1,2-a]pyrazol-1-on
2,3-Diamino-5,8-dihydro-1H,6H-pyridazino[1,2-a]pyrazol-1-on.

13. Zusammensetzung nach einem der Ansprüche 1 bis 12, bei der die Verbindung der Formel (I) oder eines ihrer Additionssalze ausgewählt ist unter:
2,3-Diamino-6,7-dihydro-1H,5H-pyrazolo[1,2-a]pyrazol-1-on
2-Amino-3-isopropylamino-6,7-dihydro-1H,5H-pyrazolo[1,2-a]-pyrazol-1-on
2-Amino-3-ethylamino-6,7-dihydro-1H,5H-pyrazolo[1,2-a]-pyrazol-1-on
2-Amino-3-(2-hydroxyethyl)amino-6,7-dihydro-1H,5H-pyrazolo[1,2-a]pyrazol-1-on
2-Amino-3-dimethylamino-6,7-dihydro-1H,5H-pyrazolo[1,2-a]-pyrazol-1-on
2-Amino-3-(pyrrolidin-1-yl)-6,7-dihydro-1H,5H-pyrazolo[1,2-a]-pyrazol-1-on
2,3-Diamino-5,6,7,8-tetrahydro-1H,6H-pyridazino[1,2-a]pyrazol-1-on.

14. Zusammensetzung nach einem der vorhergehenden Ansprüche, die ferner einen Kuppler enthält, der unter den meta-Phenylendiaminen, meta-Aminophenolen, meta-Dihydroxybenzolen, Naphthalinkupplern, heterocyclischen Kupplern und ihren Additionssalzen sowie deren Gemischen ausgewählt ist.

15. Zusammensetzung nach Anspruch 14, bei der der Mengenanteil jedes Kupplers im Bereich von 0,001 bis 10 Gew.-% des Gesamtgewichts der Farbmittelzusammensetzung liegt.

16. Zusammensetzung nach einem der vorhergehenden Ansprüche, die eine ergänzende Oxidationsbase enthält, die unter den paraPhenylendiaminen, Bisphenylalkylendiaminen, para-Aminophenolen, Bis-para-aminophenolen, ortho-Aminophenolen ortho-Phenylendiaminen und den heterocyclischen Basen, die von den in einem der Ansprüche 1 bis 13 definierten Derivaten der Formel (I) und ihren Additionssalzen verschieden sind, sowie deren Gemischen ausgewählt ist.

17. Zusammensetzung nach Anspruch 16, bei der der Mengenanteil jeder Oxidationsbase im Bereich von 0,001 bis 10 Gew.-% des Gesamtgewichts der Farbmittelzusammensetzung liegt.

18. Verfahren zum Färben von Keratinfasern, **dadurch gekennzeichnet, dass** eine Zusammensetzung, wie sie in einem der Ansprüche 1 bis 17 definiert ist, in Gegenwart eines Oxidationsmittels während einer Zeitspanne, die zur Bildung der gewünschten Färbung ausreichend ist, auf die Keratinfasern aufgebracht wird.

19. Verfahren nach Anspruch 18, wobei das Oxidationsmittel unter Wasserstoffperoxid, Harnstoffperoxid, Alkalimetallbromaten, Salzen von Persäuren, Persäuren und Oxidasen ausgewählt ist.

20. Vorrichtung mit mehreren Abteilungen, bei der eine erste Abteilung eine Farbmittelzusammensetzung, wie sie in den Ansprüchen 1 bis 17 definiert ist, und eine zweite Abteilung ein Oxidationsmittel enthält.

21. Verwendung einer Zusammensetzung, wie sie in den Ansprüchen 1 bis 17 definiert ist, zum oxidativen Färben von Keratinfasern.

22. Amino-N,N-dihydro-pyrazolonderivate der Formel (I') oder ihre Additionssalze: 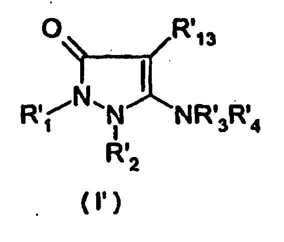 in der Formel:
R'₁ und R'₂ bilden mit den Stickstoffatomen, an die sie gebunden sind, einen gesättigten oder ungesättigten, 5- bis 7-gliedrigen Heterocyclus, der gegebenenfalls mit einer oder mehreren Gruppen substituiert ist, die ausgewählt sind unter den Halogenatomen, den Gruppen Amino, (Di)alkyl(C₁₋₄)amino, Hydroxy, Carboxy, Carboxamido, C₁₋₂-Alkoxy, den C₁₋₄-Alkylgruppen, die gegebenenfalls mit einer oder mehreren Gruppen Hydroxy, Amino, (Di)alkylamino, Alkoxy, Carboxy, Sulfonyl substituiert sind;
R'₃ et R'₄, die gleich oder verschieden sind, bedeuten:
- eine geradkettige oder verzweigte C₁₋₆-Alkylgruppe, die gegebenenfalls mit einer oder mehreren Gruppen substituiert ist, die ausgewählt sind unter einer Gruppe OR'₅, einer Gruppe NR'₆R'₇, einer Carboxygruppe, einer Sulfonsäuregruppe, einer Carboxamidogruppe CONR'₆R'₇, einer Sulfonamidogruppe SO₂NR'₆R'₇ einer Heteroarylgruppe, einer Arylgruppe, die gegebenenfalls substituiert ist mit (C₁₋₄)Alkyl, Hydroxy, (C₁-₂)-Alkoxy, Amino, (Di)alkyl(C₁₋₂)amino;
- eine Arylgruppe , die gegebenenfalls mit einer oder mehreren Gruppen C₁₋₄-Alkyl, Hydroxy, C₁₋₂-Alkoxy, Amino, (Di)alkyl(C₁₋₂) amino substituiert ist;
- eine 5- oder 6-gliedrige Heteroarylgruppe, die gegebenenfalls mit einer oder mehreren Gruppen substituiert ist, die unter C₁₋₄-Alkyl, C₁₋₂-Alkoxy ausgewählt sind;
R'₃ et R'₄ können auch ein Wasserstoffatom bedeuten ;
R'₃ et R'₄ können mit dem Stickstoffatom, an das sie gebunden sind, einen gesättigten oder ungesättigten, 5- bis 7-gliedrigen Ring bilden, der gegebenenfalls mit einer oder mehreren Gruppen substituiert ist, die ausgewählt sind unter den Halogenatomen, den Gruppen Amino, (Di)alkyl(C₁₋₄)amino, Hydroxy, Carboxy, Carboxamido, (C₁₋₂)Alkoxy, den C₁₋₄-Alkylgruppen, die gegebenenfalls mit einer oder mehreren Gruppen Hydroxy, Amino, (Di)alkylamino, Alkoxy, Carboxy, Sulfonyl substituiert sind;
R'₃ et R'₄ können auch gemeinsam mit dem Stickstoffatom, an das sie gebunden sind, einen 5- oder 7-gliedrigen Heterocyclus bilden, bei dem die Kohlenstoffatome durch ein Sauerstoffatom oder Stickstoffatom ersetzt sein können, das gegebenenfalls substituiert ist;
R'₅, R'₆ et R'₇, die gleich oder verschieden sind, bedeuten ein Wasserstoffatom; eine geradkettige oder verzweigte C₁₋₄-Alkylgruppe, die gegebenenfalls mit einer oder mehreren Gruppen substituiert ist, die ausgewählt sind unter Hydroxy, C₁₋₂-Alkoxy, Carboxamido CONR'₈R'₉, Sulfonyl SO₂R'₈, einer Arylgruppe, die gegebenenfalls substituiert ist mit (C₁₋₄)Alkyl, Hydroxy, C₁₋₂-Alkoxy, Amino, (Di)alkyl(C₁₋₂)amino; eine Arylgruppe, die gegebenenfalls substituiert ist mit C₁₋₄-Alkyl, Hydroxy, C₁₋₂-Alkoxy, Amino, (Di)alkyl(C₁₋₂)amino;
R'₆ et R'₇, die gleich oder verschieden sind, können auch eine Carboxamidogruppe CONR'₈R'₉, eine Sulfonylgruppe SO₂R'₈ bedeuten;
R'₈ et R'₉, die gleich oder verschieden sind, bedeuten ein Wasserstoffatom; eine geradkettige oder verzweigte C₁₋₄-Alkylgruppe, die gegebenenfalls mit einer oder mehreren Gruppen Hydroxy, C₁₋₂-Alkoxy substituiet ist;
R'₁₃ bedeutet eine Gruppe Nitro, Nitroso oder Arylazo Ar-N=N-, wobei die Arylgruppe Ar gegebenenfalls mit einer oder mehreren Gruppen C₁₋₄-Alkyl, Amino, (Di)alkyl(C₁₋₂)amino, C₁₋₂-Alkoxy, Sulfo, Carboxy, Halogen substituiert ist;
mit der Maßgabe, dass
R'₁₃ keine Gruppe Ar-N=N- bedeutet, wenn die Gruppen R'₃ et R'₄ gleichzeitig ein Wasserstoffatom bedeuten.

23. Diamino-N,N-dihydro-pyrazolonderivate der Formel (I") oder ihre Additionssalze: 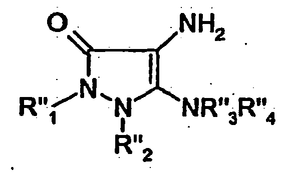 in der Formel:
R"₁ und R"₂ bilden mit den Stickstoffatomen, an die sie gebunden sind, einen gesättigten oder ungesättigten, 5- bis 7- gliedrigen Heterocyclus, der gegebenenfalls mit einer oder mehreren Gruppen substituiert ist, die ausgewählt sind unter den Halogenatomen, den Gruppen Amino, (Di)alkyl(C₁₋₄)amino, Hydroxy, Carboxy, Carboxamido, C₁₋₂-Alkoxy, den C₁₋₄-Alkylgruppen, die gegebenenfalls mit einer oder mehreren Gruppen Hydroxy, Amino, (Di)alkylamino, Alkoxy, Carboxy, Sulfonyl substituiert sind;
R"₃ et R"₄, die gleich oder verschieden sind, bedeuten:
- eine geradkettige oder verzweigte C₁₋₆-Alkylgruppe, die gegebenenfalls mit einer oder mehreren Gruppen substituiert ist, die ausgewählt sind unter einer Gruppe OR"₅, einer Gruppe NR"₆R"₇, einer Carboxygruppe, einer Sulfonsäuregruppe, einer Carboxamidogruppe CONR"₆R"₇, einer Sulfonamidogruppe SO₂NR"₆R"₇, einer Heteroarylgruppe, einer Arylgruppe, die gegebenenfalls substituiert ist mit C₁₋₄-Alkyl, Hydroxy, C₁₋₂-Alkoxy, Amino, (Di)alkyl(C₁₋₂)amino;
- eine Arylgruppe, die gegebenenfalls mit einer oder mehreren Gruppen C₁₋₄-Alkyl, Hydroxy, C₁₋₂-Alkoxy, Amino, (Di)alkyl-(C₁₋₂)amino substituiert ist;
- eine 5- oder 6-gliedrige Heteroarylgruppe, die gegebenenfalls mit einer oder mehreren Gruppen C₁₋₄-Alkyl, C₁₋₂-Alkoxy substituiert ist;
R"₃ et R"₄ können auch ein Wasserstoffatom bedeuten;
R"₅, R"₆ et R"₇, die gleich oder verschieden sind, bedeuten ein Wasserstoffatom; eine geradkettige oder verzweigte C₁₋₄-Alkylgruppe, die gegebenenfalls mit einer oder mehreren Gruppen substituiert ist, die ausgewählt sind unter Hydroxy, C₁₋₂-Alkoxy, Carboxamido CONR"₈R"₉, Sulfonyl SO₂R"₈, einer Arylgruppe, die gegebenenfalls substituiert ist mit C₁₋₄-Alkyl, Hydroxy, C₁₋₂-Alkoxy, Amino, (Di)alkyl(C₁₋₂)amino; eine Arylgruppe, die gegebenenfalls substituiert ist mit C₁₋₄-Alkyl, Hydroxy, C₁₋₂-Alkoxy, Amino, (Di)alkyl(C₁₋₂)amino;
R"₆ et R"₇, die gleich oder verschieden sind, können auch eine Carboxamidogruppe CONR"₈R"₉, eine Sulfonylgruppe SO₂R"s bedeuten;
R"₈ et R"₉, die gleich oder verschieden sind, bedeuten ein Wasserstoffatom; eine geradkettige oder verzweigte C₁₋₄-Alkylgruppe, die gegebenenfalls mit einer oder mehreren Gruppen Hydroxy, C₁₋₂-Alkoxy substituiert ist;
R"₃ et R"₄ können mit dem Stickstoffatom, an das sie gebunden sind, einen gesättigten oder ungesättigten, 5- bis 7-gliedrigen Heterocyclus bilden, der gegebenenfalls mit einer oder mehreren Gruppen substituiert ist, die ausgewählt sind unter den Halogenatomen, den Gruppen Amino, (Di)alkyl(C₁-₄)amino, Hydroxy, Carboxy, Carboxamido, (C₁₋₂)Alkoxy, den C₁₋₄-Alkylgruppen, die gegebenenfalls mit einer oder mehreren Gruppen Hydroxy, Amino, (Di)alkylamino, Alkoxy, Carboxy, Sulfonyl substituiert sind;
R"₃ et R"₄ können auch gemeinsam mit dem Stickstoffatom, an das sie gebunden sind, einen 5- oder 7-gliedrigen Heterocyclus bilden, bei dem die Kohlenstoffatome durch ein Sauerstoffatom oder Stickstoffatom ersetzt sein können, das gegebenenfalls substituiert ist.

24. Derivate nach einem der Ansprüche 22 oder 23, bei denen unabhängig voneinander die Gruppen R"₁ und R"₂ einerseits und R'₁ und R'₂ andererseits gemeinsam mit den Stickstoffatomen, an die sie gebunden sind, einen gesättigten oder ungesättigten, 5- oder 6-gliedrigen Ring bilden, der gegebenenfalls substituiert ist.

25. Derivate nach einem der Ansprüche 22 bis 24, bei denen die Gruppen R"₁ und R"₂ einerseits und R'₁ und R'₂ andererseits gemeinsam mit den Stickstoffatomen, an die sie gebunden sind, einen Pyrazolidinring oder Pyridazolidinring bilden, die gegebenenfalls mit (C₁₋₄)Alkyl, Hydroxy, (C₁₋₂)-Alkoxy, Carboxy, Carboxamido, Amino, (Di)alkyl(C₁₋₂)amino substituiert sind.

26. Derivate nach einem der Ansprüche 22 bis 25, bei denen die Gruppen R"₁ und R"₂ einerseits und R'₁ und R'₂ andererseits gemeinsam mit den Stickstoffatomen, an die sie gebunden sind, einen Pyrazolidinring oder Pyridazolidinring bilden.

27. Derivate nach einem der Ansprüche 22 bis 26, bei denen die Gruppen R"₃, R"₄, R'₃ und R'₄ unabhängig voneinander unter einem Wasserstoffatom; einer geradkettigen oder verzweigten Alkyl(C₁₋₄)gruppe, die gegebenenfalls mit einer oder mehreren Gruppen Hydroxy, (C₁₋₂)Alkoxy, Amino, (Di)alkyl(C₁₋₂)amino substituiert ist; einer Phenylgruppe, die gegebenenfalls mit Hydroxy, Amino, (C₁₋₂)Alkoxy substituiert ist, ausgewählt sind.

28. Derivate nach einem der Ansprüche 22 bis 27, bei denen die Gruppen R"₃, R"₄, R'₃ und R'₄ unabhängig voneinander unter einem Wasserstoffatom, Methyl, Ethyl, Isopropyl, 2-Hydroxy-ethyl, 3-Hydroxypropyl, 2-Hydroxypropyl, 2-Carboxyethyl ausgewählt sind.

29. Derivate nach einem der Ansprüche 22 bis 26, bei denen die Gruppen R"₃ und R"₄ einerseits und R'₃ und R'₄ andererseits gemeinsam mit dem Stickstoffatom, an das sie gebunden sind, einen 5- oder 7-gliedrigen Ring bilden, der unter den Heterocyclen Pyrrolidin; Piperidin; Homopiperidin; Piperazin; Homopiperazin ausgewählt ist, wobei diese Ringe gegebenenfalls substituiert sein können mit einer oder mehreren Gruppen Hydroxy, Amino, (Di)alkyl(C₁-₂)amino, Carboxy, Carboxamido, (C₁-₄)Alkyl, wobei diese Gruppe gegebenenfalls mit einer oder mehreren Gruppen Hydroxy, Amino, (Di)alkyl(C₁₋₂)amino substituiert sein kann.

30. Derivate nach einem der Ansprüche 22 bis 26 und 29, bei denen die Gruppen R"₃ und R"₄ einerseits und R'₃ und R'₄ andererseits gemeinsam mit dem Stickstoffatom, an das sie gebunden sind, einen 5- oder 7-gliedrigen Ring bilden, der unter Pyrrolidin, 2,5-Dimethylpyrrolidin, Pyrrolidin-2-carbonsäure, 3-Hydroxypyrrolidin-2-carbonsäure, 4-Hydroxypyrrolidin-2-carbonsäure, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)pyrrolidin, 2-Hydroxymethyl-pyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 3-Dimethylamino-pyrrolidin, 4-Amino-3-hydroxypyrrolidin, 3-Hydroxy-4-(2-hydroxyethyl)amino-pyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxy-homo-piperidin, 2-Carboxamidohomopiperidin, Homopiperazin, N-Methyl-homopiperazin, N-(2-Hydroxyethyl)-homopiperazin ausgewählt ist.

31. Derivate nach einem der Ansprüche 22 bis 26 und 29 und 30, bei denen die Gruppen R"₃ und R"₄ einerseits und R'₃ und R'₄ andererseits gemeinsam mit dem Stickstoffatom, an das sie gebunden sind, einen 5- oder 7-gliedrigen Ring bilden, der unter Pyrrolidin, 3-Hydroxypyrrolidin, 3-Aminopyrrolidin, 3-Dimethylaminopyrrolidin, Pyrrolidin-2-carbonsäure, 3-Hydroxypyrrolidin-2-carbonsäure, Piperidin, Hydroxypiperidin, Homopiperidin, Diazepan, N-Methylhomopiperazin, N-β-Hydroxyethylhomopiperazin ausgewählt ist.

32. Derivate nach einem der Ansprüche 22 bis 26 und 29 bis 31, bei denen die Gruppen R"₃ und R"₄ einerseits und R'₃ und R'₄ andererseits gemeinsam mit dem Stickstoffatom, an das sie gebunden sind, einen 5-gliedrigen Ring bilden, der unter Pyrrolidin, 3-Hydroxypyrrolidin, 3-Aminopyrrolidin, 3-Dimethylamino-pyrrolidin ausgewählt ist.

33. Verfahren zur Herstellung einer Verbindung der Formel (I), wie sie in den Zusammensetzungen nach einem der Ansprüche 1 bis 13 definiert ist, **dadurch gekennzeichnet, dass** die folgenden Schritte durchgeführt werden:
a) Schritt 1: es wird eine Verbindung a
**R₁HN-NHR₂** **a**
mit einer Verbindung b umgesetzt: 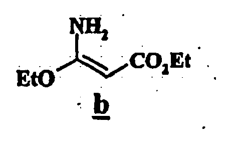 um ein 5-Amino-1,2-dihydro-pyrazol-3-on c zu bilden: 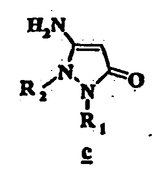
b) Schritt 2: das auf diese Weise gebildete Derivat c wird mit einem Aryldiazoniumsalz (Ar-N₂⁺Y-) umgesetzt, um eine Azoverbindung f zu bilden: 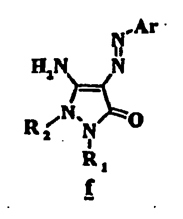
c) Schritt 3: gegebenenfalls wird die primäre Aminogruppe der resultierenden Azoverbindung f funktionalisiert, um die folgende Verbindung g zu bilden: 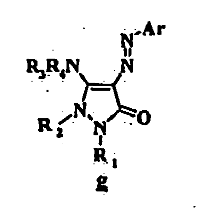
d) Schritt 4: die Azoverbindung f oder g wird reduziert, um ein Aminoderivat e oder h zu bilden:

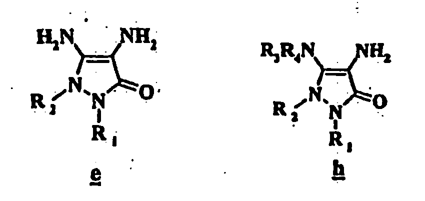

34. Verfahren zur Herstellung einer Verbindung der Formel (I), wie sie in den Zusammensetzungen nach einem der Ansprüche 1 bis 13 definiert ist, **dadurch gekennzeichnet, dass** die folgenden Schritte durchgeführt werden:
a) Schritt 1: es wird eine folgende Verbindung a1: 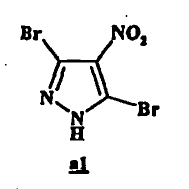 mit einer Verbindung a2 umgesetzt: 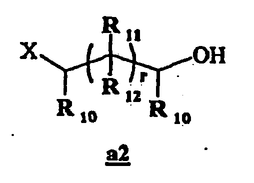 um eine Verbindung a3 zu bilden: 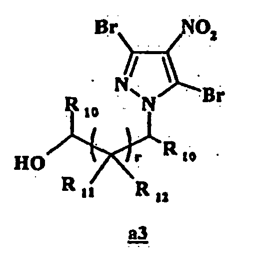 in den Formeln:
die Gruppe R₁₀ bedeutet ein Wasserstoffatom, Carboxy; Carboxamido ; eine C₁₋₄-Alkylgruppe, die gegebenenfalls mit einer oder mehreren Gruppen Hydroxy, Amino, (Di)-alkylamino, Alkoxy, Carboxy, Sulfonyl substituiert ist;
die Gruppen R₁₁ et R₁₂ bedeuten unabhängig voneinander Wasserstoffatome, Halogenatome; Gruppen Amino; (Di)alkyl-(C₁₋₄)amino; Hydroxy; Carboxy; Carboxamido ; (C₁₋₂)Alkoxy;
eine C₁₋₄-Alkylgruppe, die gegebenenfalls mit einer oder mehreren Gruppen Hydroxy, Amino, (Di)-alkylamino, Alkoxy, Carboxy, Sulfonyl substituiert ist;
X bedeutet ein Halogenatom oder eine Alkylsulfonatgruppe;
r ist eine ganze Zahl im Bereich von 1 bis 3 ;
b) Schritt 2: die Verbindung a3 wird mit einem Amin der Formel NHR₃R₄ umgesetzt, um eine Verbindung a4 zu bilden: 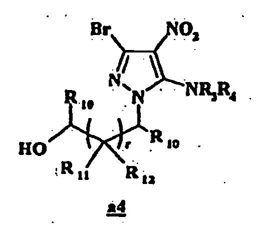
c) Schritt 3: die Verbindung a4 wird mit mindestens einem Alkylsulfonylhalogenid, Arylsulfonylhalogenid oder Perfluoralkylsulfonylhalogenid R-O₂S-X₁ (R bedeutet Alkyl, Aryl oder Perfluoralkyl, X₁ ist ein Halogen) in einem aprotischen Lösemittel umgesetzt, um eine Verbindung a5 zu bilden: 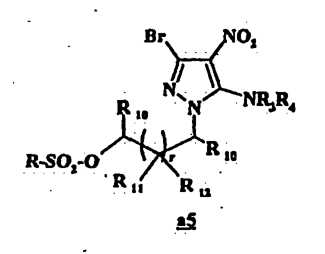
d) Schritt 4: die resultierende Verbindung a5 wird dann in einem Lösemittel mit einem Siedepunkt im Bereich von 60 bis 190 °C erwärmt, um eine Verbindung a6 zu bilden: 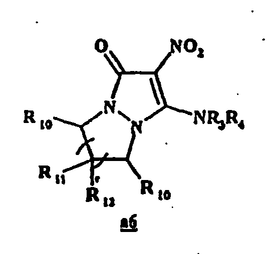
e) Schritt 5: die erhaltene Verbindung a6 wird reduziert, um eine Verbindung der Formel (III) zu bilden:
